(19) Europäisches Patentamt
European Patent Office
Office européen des brevets

(11) Veröffentlichungsnummer: **0 242 573**
**A1**

(12) **EUROPÄISCHE PATENTANMELDUNG**

(21) Anmeldenummer: **87103584.6**

(22) Anmeldetag: **12.03.87**

(51) Int.Cl.³: **C 07 D 405/12**
C 07 D 409/12, C 07 D 403/12
C 07 D 413/12, C 07 D 417/12
C 07 D 401/12, C 07 D 411/12
A 01 N 43/56, A 01 N 43/08

(30) Priorität: **21.03.86 DE 3609542**

(43) Veröffentlichungstag der Anmeldung:
**28.10.87 Patentblatt 87/44**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Stetter, Jörg, Dr.**
**Gellertweg 4**
**D-5600 Wuppertal 1(DE)**

(72) Erfinder: **Gehring, Reinhold, Dr.**
**Dasnöckel 49**
**D-5600 Wuppertal 11(DE)**

(72) Erfinder: **Schallner, Otto, Dr.**
**Noldeweg 22**
**D-4019 Monheim(DE)**

(72) Erfinder: **Santel, Hans-Joachim, Dr.**
**Grünstrasse 9a**
**D-5090 Leverkusen 1(DE)**

(72) Erfinder: **Schmidt, Robert R., Dr.**
**Im Waldwinkel 110**
**D-5060 Bergisch Gladbach(DE)**

(72) Erfinder: **Lürssen, Klaus, Dr.**
**August-Kierspel-Strasse 151**
**D-5060 Bergisch Gladbach 2(DE)**

(54) **5-Acylamino-pyrazol-Derivate.**

(57) Die Erfindung betrifft 5-Acylamino-pyrazol-Derivate der allgemeinen Formel (I),

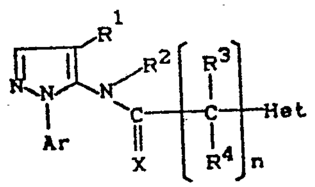

(I)

in welcher

$R^1$ für Wasserstoff, Halogen oder Nitro steht,

$R^2$ für Wasserstoff, Alkyl, Alkenyl, Alkinyl oder gegebenenfalls substituiertes Cycloalkyl steht,

$R^3$ für Wasserstoff oder Alkyl steht,

$R^4$ für Wasserstoff oder Alkyl steht,

X für Sauerstoff oder Schwefel steht,

n für die ganzen Zahlen 0, 1 oder 2 steht,

Ar für jeweils gegebenenfalls substituiertes Phenyl oder Pyridyl steht und

Het für einen gegebenenfalls substituierten 5- oder 6-gliedrigen, über ein Kohlenstoffatom verknüpften Heterocyclus steht,

mehrere Verfahren zu ihrer Herstellung und ihre Verwendung als Herbizide und Wachstumsregulatoren.

EP 0 242 573 A1

BAYER AKTIENGESELLSCHAFT
Konzernverwaltung RP
Patentabteilung

5090 Leverkusen, Bayerwerk

KM/cm/c
Ib

# 5-Acylamino-pyrazol-Derivate

Die Erfindung betrifft neue 5-Acylamino-pyrazol-Derivate, mehrere Verfahren zu ihrer Herstellung sowie ihre Verwendung als Herbizide und Wachstumsregulatoren.

Es ist bereits bekannt, daß bestimmte 5-Acylamino-1-aryl-pyrazole, wie beispielsweise das 4-Cyano-5-propionamido-1-(2,4,6-trichlorphenyl)-pyrazol (vgl. DE-OS 32 26 513) herbizide Eigenschaften besitzen.

Die herbizide Wirksamkeit dieser vorbekannten Verbindungen gegenüber Unkräutern ist jedoch ebenso wie ihre Verträglichkeit gegenüber wichtigen Kulturpflanzen nicht immer in allen Anwendungsbereichen völlig zufriedenstellend.

Es wurden neue 5-Acylamino-pyrazol-Derivate der allgemeinen Formel (I),

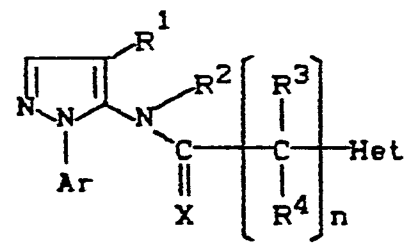

(I)

in welcher

$R^1$ für Wasserstoff, Halogen oder Nitro steht,

$R^2$ für Wasserstoff, Alkyl, Alkenyl, Alkinyl oder gegebenenfalls substituiertes Cycloalkyl steht,

$R^3$ für Wasserstoff oder Alkyl steht,

$R^4$ für Wasserstoff oder Alkyl steht,

X für Sauerstoff oder Schwefel steht,

n für die ganzen Zahlen 0, 1 oder 2 steht,

Ar für jeweils gegebenenfalls substituiertes Phenyl oder Pyridyl steht und

Het für einen gegebenenfalls substituierten 5- oder 6-gliedrigen, über ein Kohlenstoffatom verknüpften Heterocyclus steht,

gefunden.

Weiterhin wurde gefunden, daß man die neuen 5-Acylamino-pyrazol-Derivate der Formel (I)

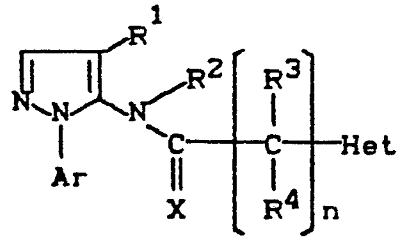 (I)

in welcher

$R^1$ für Wasserstoff, Halogen oder Nitro steht,

$R^2$ für Wasserstoff, Alkyl, Alkenyl, Alkinyl oder gegebenenfalls substituiertes Cycloalkyl steht,

$R^3$ für Wasserstoff oder Alkyl steht,

$R^4$ für Wasserstoff oder Alkyl steht,

X für Sauerstoff oder Schwefel steht,

n für die ganzen Zahlen 0, 1 oder 2 steht,

Ar für jeweils gegebenenfalls substituiertes Phenyl oder Pyridyl steht und

Het für einen gegebenenfalls substituierten 5- oder 6-gliedrigen, über ein Kohlenstoffatom verknüpften Heterocyclus steht,

mit Hilfe der im folgenden beschriebenen Verfahren erhält:

a) Man erhält 5-Acylamino-pyrazol-Derivate der Formel (Ia)

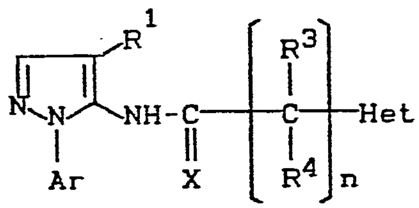

(Ia)

in welcher

$R^1$, $R^3$, $R^4$, X, Ar, Het und der Index n die oben angegebene Bedeutung haben,

wenn man 5-Amino-1-aryl-pyrazole der Formel (II)

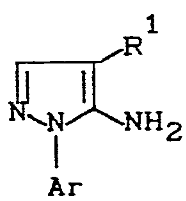

(II)

in welcher

$R^1$ und Ar die oben angegebene Bedeutung haben,

mit Acylierungsmitteln der Formel (III)

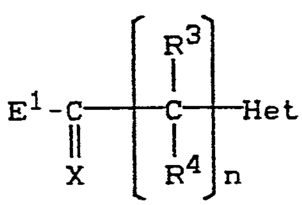

(III)

in welcher

X, $R^3$, $R^4$, Het und der Index n die oben angegebene Bedeutung haben und

$E^1$ für eine elektronenanziehende Abgangsgruppe steht,

gegebenenfalls in Gegenwart eines Verdünnungsmittels, gegebenenfalls in Gegenwart eines Säurebindemittels sowie gegebenenfalls in Gegenwart eines Katalysators umsetzt;

b) Man erhält 5-Acylamino-pyrazol-Derivate der Formel (Ib)

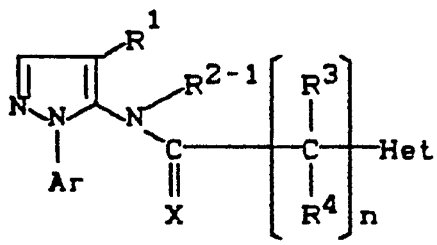

(Ib)

in welcher

$R^1$, $R^3$, $R^4$, X, Ar, Het und der Index n die oben angegebene Bedeutung haben und

$R^{2-1}$ für Alkyl, Alkenyl, Alkinyl oder gegebenenfalls substituiertes Cycloalkyl steht,

wenn man die nach Verfahren (a) erhältlichen 5-Acylamino-pyrazol-Derivate der Formel (Ia)

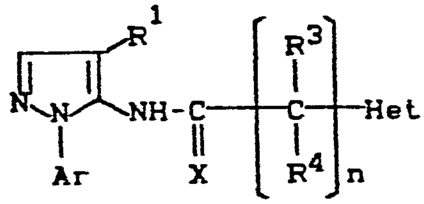 (Ia)

in welcher

$R^1$,$R^3$,$R^4$,X,Ar,Het und der Index n die oben angegebene Bedeutung haben,

mit Alkylierungsmitteln der Formel (IV)

$E^2$-$R^{2-1}$ (IV)

in welcher

$R^{2-1}$ die oben angegebene Bedeutung hat und

$E^2$ für Halogen, für gegebenenfalls substituiertes Alkoxysulfonyloxy oder für gegebenenfalls substituiertes Arylsulfonyloxy steht,

gegebenenfalls in Gegenwart eines Verdünnungsmittel und gegebenenfalls in Gegenwart eines Säurebindemittels sowie gegebenenfalls in Gegenwart eines Katalysators umsetzt;

c) Man erhält 5-Acylamino-pyrazol-Derivate der Formel (Ic)

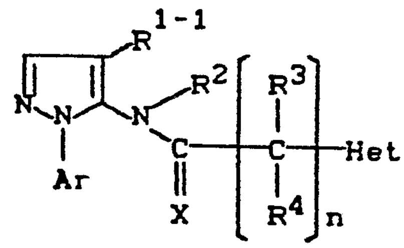

(Ic)

in welcher

$R^2$,$R^3$,$R^4$,X,Ar,Het und der Index n die oben angegebene Bedeutung haben und

$R^{1-1}$ für Halogen oder Nitro steht,

alternativ auch, wenn man die mit Hilfe der Verfahren (a) oder (b) erhältlichen 5-Acylamino-pyrazol-Derivate der Formel (Id)

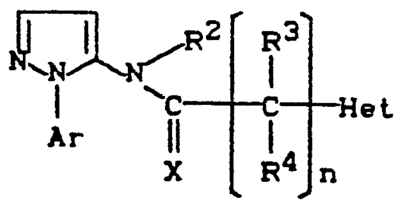

(Id)

in welcher

$R^2$,$R^3$,$R^4$,X,Ar,Het und der Index n die oben angegebene Bedeutung haben,

mit Halogenierungs- oder Nitrierungsmitteln der Formel (V)

$$R^{1-1}-E^3 \quad (V)$$

in welcher

$R^{1-1}$ die oben angegebene Bedeutung hat und

$E^3$ für eine elektronenanziehende Abgangsgruppe steht,

gegebenenfalls in Gegenwart eines Verdünnungsmittels und gegebenenfalls in Gegenwart eines Katalysators oder Reaktionshilfsmittels umsetzt.

Schließlich wurde gefunden, daß die neuen 5-Acylamino-pyrazol-Derivate der allgemeinen Formel (I) herbizide, insbesondere auch selektiv-herbizide Eigenschaften sowie pflanzenwachstumsregulierende Eigenschaften besitzen.

Überraschenderweise zeigen die erfindunsggemäßen 5-Acylamino-pyrazol-Derivate der allgemeinen Formel (I) eine erheblich bessere allgemein-herbizide Wirksamkeit gegen schwer bekämpfbare Problemunkräuter und gleichzeitig eine deutlich verbesserte Verträglichkeit gegenüber wichtigen Kulturpflanzen, wie insbesondere Soja und Weizen, im Vergleich zu den aus dem Stand der Technik bekannten 5-Acylamido-1-aryl-pyrazolen, wie beispielsweise 4-Cyano-5-propionamido-1-(2,4,6-trichlorphenyl)-pyrazol, welches chemisch und wirkungsmäßig eine naheliegende Verbindung ist.

Die erfindungsgemäßen 5-Acylamino-pyrazol-Derivate sind durch die Formel (I) allgemein definiert. Bevorzugt sind Verbindungen der Formel (I), bei welchen

$R^1$ für Wasserstoff, Nitro, Fluor, Chlor, Brom oder Iod steht;

$R^2$ für Wasserstoff, für geradkettiges oder verzweigtes Alkyl mit 1 bis 12 Kohlenstoffatomen, für geradkettiges oder verzweigtes Alkenyl und Alkinyl mit jeweils 3 bis 8 Kohlenstoffatomen, sowie für gegebenenfalls einfach oder zweifach, gleich oder verschieden substituiertes Cycloalkyl mit 3 bis 8 Kohlenstoffatomen steht, wobei als Substituenten Halogen und Alkyl mit 1 bis 4 Kohlenstoffatomen genannt seien;

$R^3$ für Wasserstoff oder für geradkettiges oder verzweigtes Alkyl mit 1 bis 6 Kohlenstoffatoemn steht;

$R^4$ für Wasserstoff oder für geradkettiges oder verzweigtes Alkyl mit 1 bis 6 Kohlenstoffatomen steht;

X für Sauerstoff oder Schwefel steht;

n für die ganzen Zahlen 0, 1 oder 2 steht;

Ar für gegebenenfalls einfach oder mehrfach, gleich oder verschieden substituiertes Phenyl oder für jeweils gegebenenfalls einfach oder mehrfach, gleich oder verschieden substituiertes 2-Pyridyl, 3-Pyridyl oder 4-Pyridyl steht, wobei als Phenyl- und Pyridyl-Substituenten in Frage kommen: Cyano, Nitro, Halogen, jeweils

geradkettiges oder verzweigtes Alkyl, Alkoxy oder Alkoxycarbonyl mit jeweils 1 bis 4 Kohlenstofftomen im Alkylteil, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^5$,

wobei

$R^5$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

m für die ganzen Zahlen 0, 1 oder 2 steht;

Het für einen gegebenenfalls einfach bis dreifach, gleich oder verschieden substituierten 5- oder 6-gliedrigen, über ein Kohlenstoffatom verknüpften, ungesättigten, teilweise gesättigten oder gesättigten Heterocyclus mit ein bis drei gleichen oder verschiedenen Heteroatomen (Sauerstoff, Stickstoff, Schwefel) steht, wobei als Substituenten genannt seien: Halogen, Alkyl, Alkoxy und Alkylthio mit jeweils 1 bis 4 Kohlenstoffatomen, sowie Halogenalkyl, Halogenalkoxy und Halogenalkylthio mit jeweils 1 bis 2 Kohlenstoffatomen und 1 bis 5 gleichen oder verschiedenen Halogenatomen, wie vorzugsweise Fluor und Chlor.

Vorzugsweise seien im einzelnen die folgenden Heterocyclen genannt:

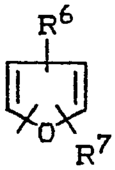
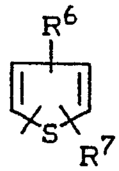
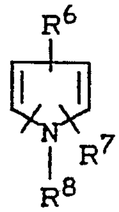
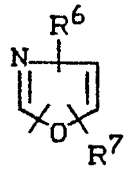
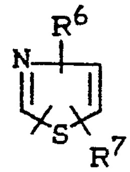

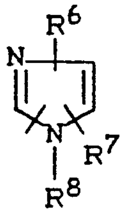
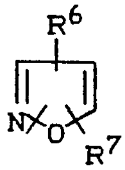
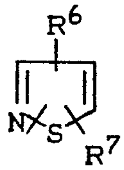
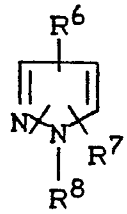
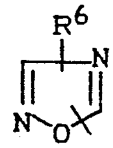

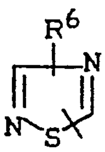
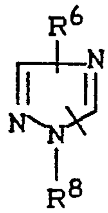
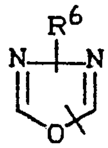
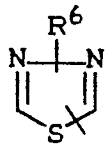
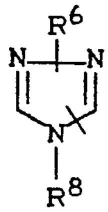

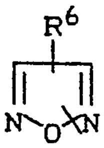
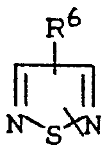
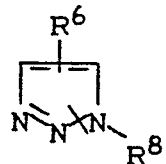
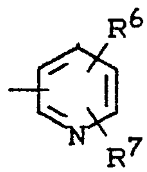
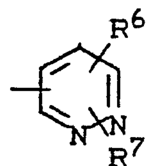

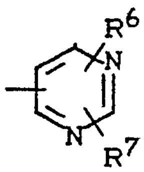
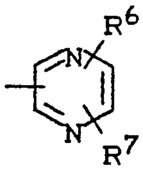
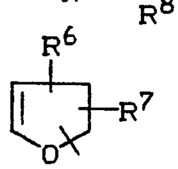
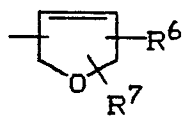
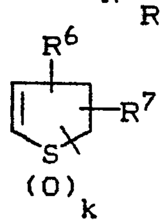

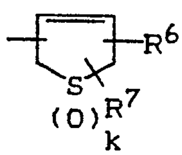
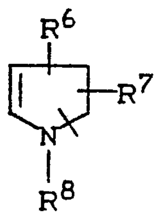
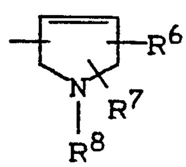

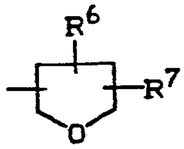 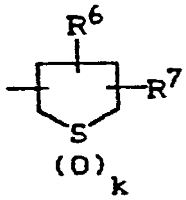 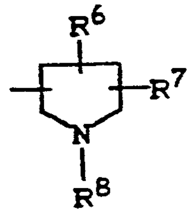 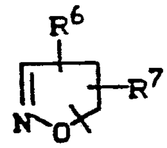

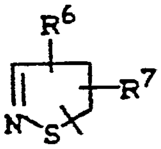 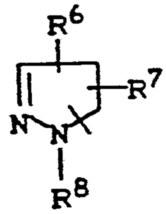 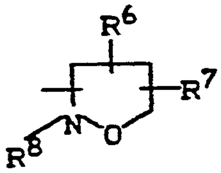 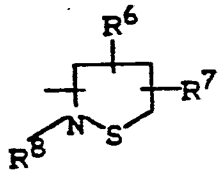

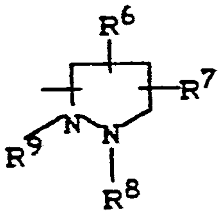 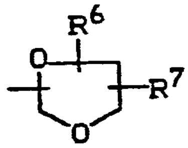 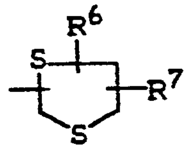 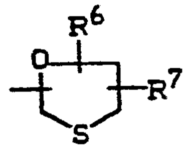

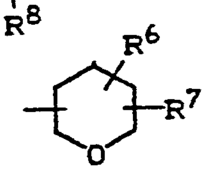 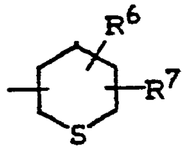 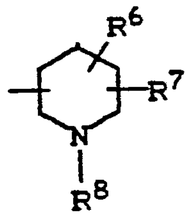 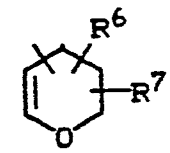

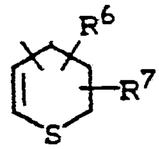 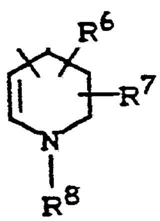 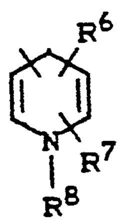 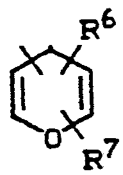 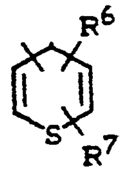

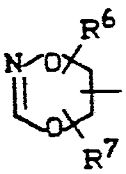 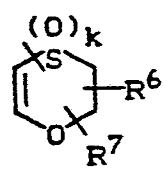 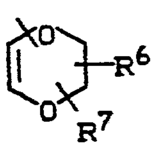 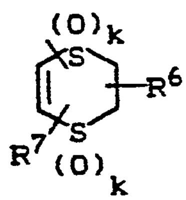 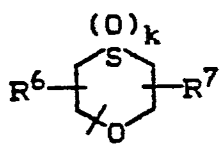

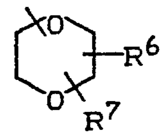 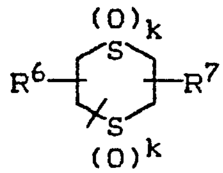 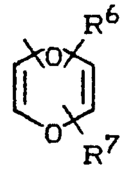

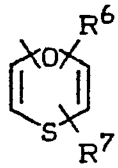 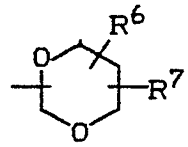 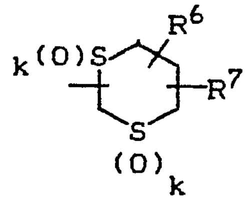

wobei in diesen Heterocyclen vorzugsweise

$R^6$ und $R^7$ unabhängig voneinander für Wasserstoff, Halogen, Alkyl, Alkoxy und Alkylthio mit jeweils 1 bis 4 Kohlenstoffatomen; sowie Halogenalkyl, Halogenalkoxy und Halogenalkylthio mit jeweils 1 bis 2 Kohlenstoffatomen und 1 bis 5 gleichen oder verschiedenen Halogenatomen, wie insbesondere Fluor und Chlor, stehen;

$R^8$ für Wasserstoff oder Alkyl mit 1 bis 4 Kohlenstoffatomen steht und

k für die ganzen Zahlen 0,1 oder 2 steht.

Besonders bevorzugt sind 5-Acylamino-pyrazol-Derivate der Formel (I), bei denen

$R^1$ für Wasserstoff, Nitro, Chlor oder Brom steht;

$R^2$ für Wasserstoff, für geradkettiges oder verzweigtes Alkyl mit 1 bis 8 Kohlenstoffatomen, sowie für geradkettiges oder verzweigtes Alkenyl und Alkinyl mit jeweils 3 bis 6 Kohlenstoffatomen steht;

$R^3$ für Wasserstoff oder Methyl steht;

$R^4$ für Wasserstoff oder Methyl steht;

X für Sauerstoff oder Schwefel steht;

n für die ganzen Zahlen 0 oder 1 steht;

Ar für gegebenenfalls ein- bis fünffach, gleich oder verschieden substituiertes Phenyl oder für jeweils gegebenenfalls ein- bis vierfach, gleich oder verschieden substituiertes 2-Pyridyl, 3-Pyridyl oder 4-Pyridyl steht, wobei als Phenyl- und Pyridyl-Substijeweils in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Difluorchlorethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^5$,

wobei

$R^5$ für Amino, Methylamino, Ethylamino, Dimethylamino, Diethylamino, Fluordichlormethyl, Difluormethyl, Tetrafluorethyl, Trifluorchlorethyl, Trichlorethyl, Trichlormethyl, Trifluormethyl, Methyl oder Ethyl steht,

und

m für die ganzen Zahlen 0, 1 oder 2 steht,

Het für einen gegebenenfalls einfach bis dreifach, gleich oder verschieden substituierten 5- oder 6-gliedrigen, über ein Kohlenstoffatom verknüpften, ungesättigten, teilweise gesättigten oder gesättigten Heterocyclus mit ein bis drei gleichen oder verschiedenen Heteroatomen (Sauerstoff, Stickstoff, Schwefel) steht, wobei als Substituenten genannt seien: Chlor, Brom, Methyl, Methoxy, Methylthio und Trifluormethyl.

Besonders bevorzugt seien im einzelnen die folgenden Heterocyclen genannt:

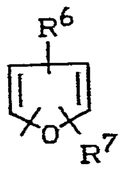 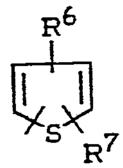 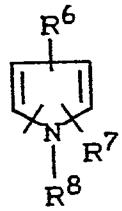 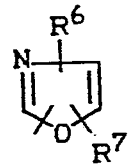 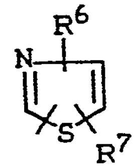

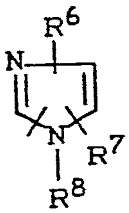 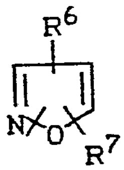 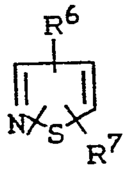 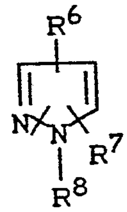 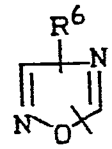

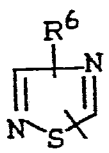 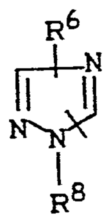 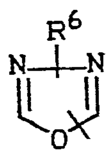 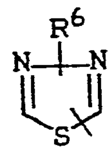 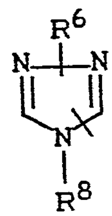

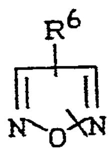 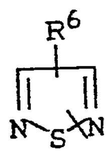 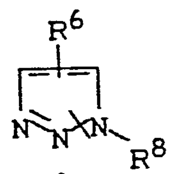 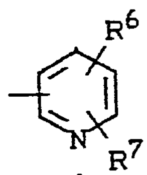 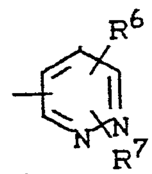

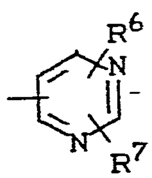 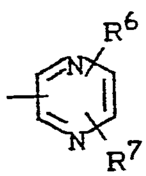 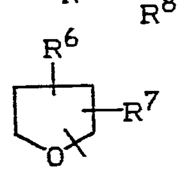 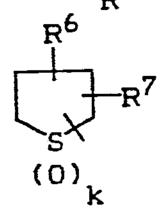 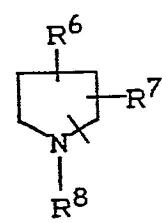

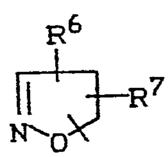 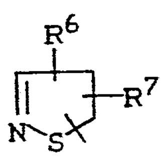 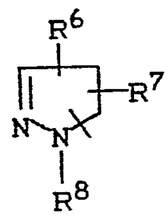 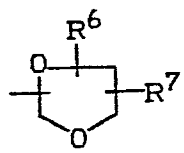 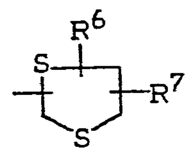

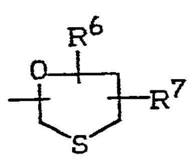 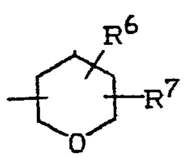 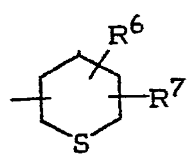 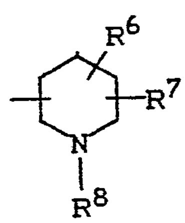 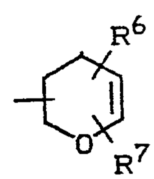

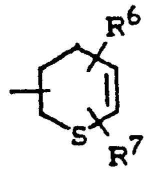 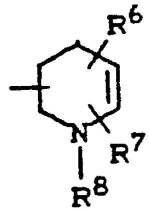 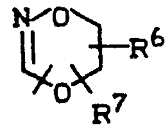 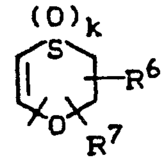 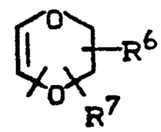

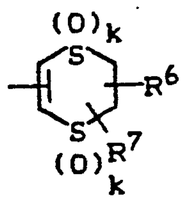 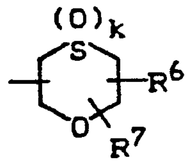 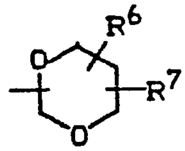 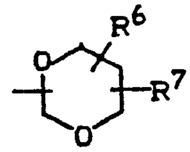 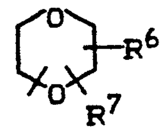

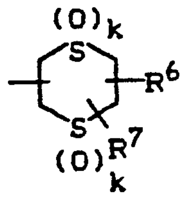

wobei in diesen Heterocyclen besonders bevorzugt

$R^6$ und $R^7$ unabhängig voneinander für Wasserstoff, Chlor, Brom, Methyl, Methoxy, Methylthio oder Trifluormethyl stehen;

$R^8$ für Wasserstoff oder Methyl steht und

k für die ganzen Zahlen 0, 1 oder 2 steht.

Ganz besonders bevorzugt sind 5-Acylamino-pyrazol-Derivate der Formel (I), bei denen

$R^1$ für Wasserstoff oder Nitro steht;

$R^2$ für Wasserstoff, Methyl, Ethyl, Allyl oder Propargyl steht;

$R^3$ für Wasserstoff steht;

$R^4$ für Wasserstoff steht;

X für Sauerstoff oder Schwefel steht;

n für die ganzen Zahlen 0 oder 1 steht;

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^5$,

wobei

$R^5$ für Amino, Methylamino, Ethylamino, Dimethylamino, Diethylamino, Fluordichlormethyl, Difluormethyl, Tetrafluorethyl, Trichlorethyl, Trichlormethyl, Trifluormethyl, Methyl oder Ethyl steht

und

m für die ganzen Zahlen 0, 1 oder 2 steht,

Het für einen gegebenenfalls einfach bis dreifach, gleich oder verschieden durch Methyl und Chlor substituierten 5- oder 6-gliedrigen, über ein Kohlenstoffatom verknüpften, ungesättigten, gesättigten oder teilweise gesättigten Heterocyclus mit ein bis drei gleichen oder verschiedenen Heteroatomen (Sauerstoff, Stickstoff, Schwefel) steht, wobei im einzelnen die folgenden Heterocyclen genannt seien:

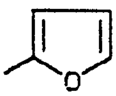 , 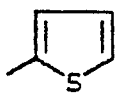 , 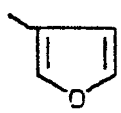 , 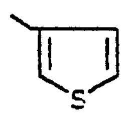 , 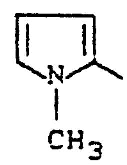 ,

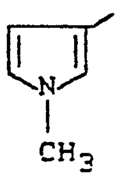 , 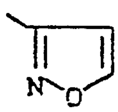 , 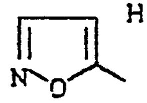 , 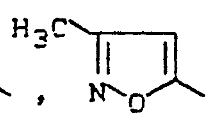 , 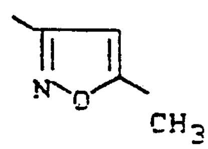 ,

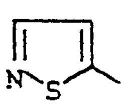 , 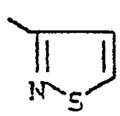 , 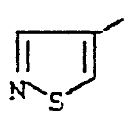 , 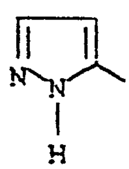 , 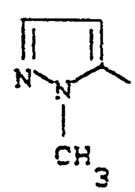

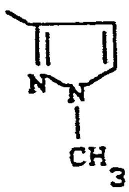 , 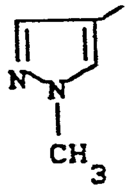 , 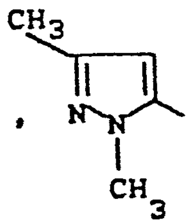 , 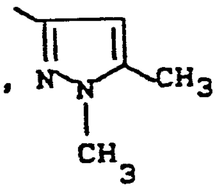 , 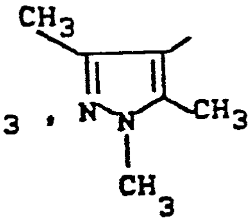

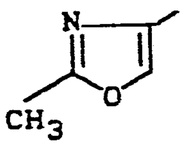 , 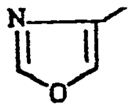 , 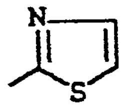 , 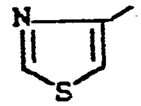 , 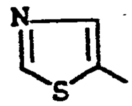 ,

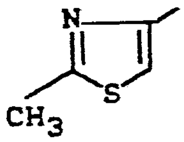 , 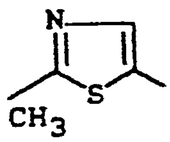 , 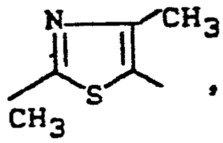 , 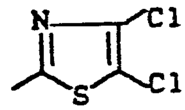 ,

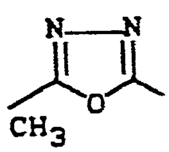 , 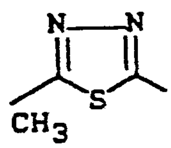 , 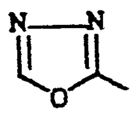 , 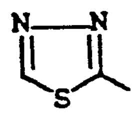 ,

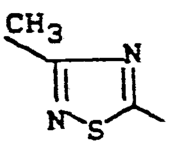 , 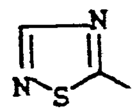 , 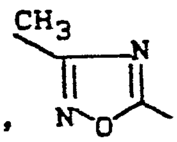 , 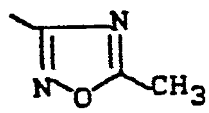 ,

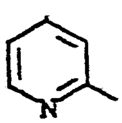 , 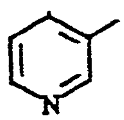 , 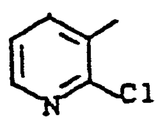 , 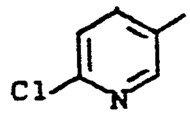 ,

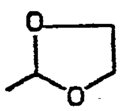 , 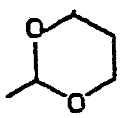 , 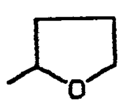 , 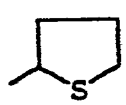 , 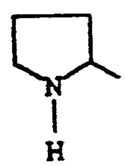 ,

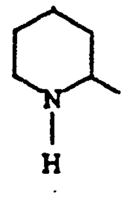 , 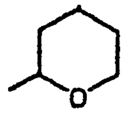 , 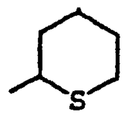 , 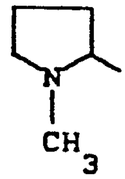 , 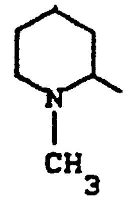 ,

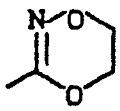 , 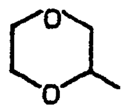 , 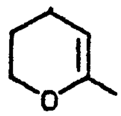 , 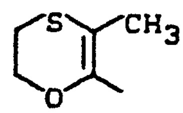 , 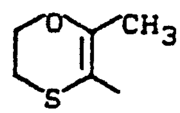 ,

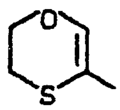 , 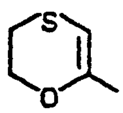 .

Im einzelnen seien außer den bei den Herstellungsbeispielen genannten Verbindungen die folgenden 5-Acylamino-pyrazol-Derivate der allgemeinen Formel (I) genannt:

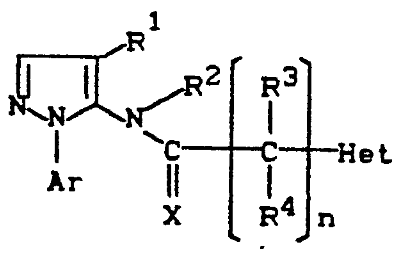 (I)

Tabelle 1

| $R^1$ | $R^2$ | $R^3$ | $R^4$ | n | X | Het | Ar |
|---|---|---|---|---|---|---|---|
| H | H | - | - | 0 | 0 | 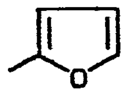 | 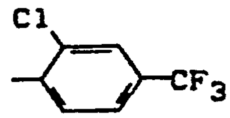 |
| $NO_2$ | H | - | - | 0 | 0 | 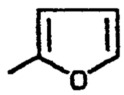 | 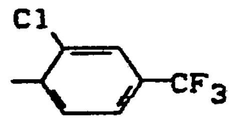 |
| Cl | H | - | - | 0 | 0 | 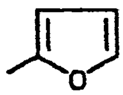 | 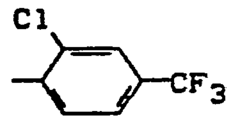 |
| Br | H | - | - | 0 | 0 | 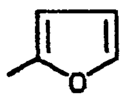 | 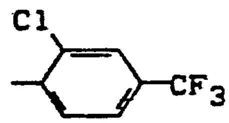 |
| H | H | - | - | 0 | 0 | 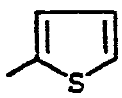 | 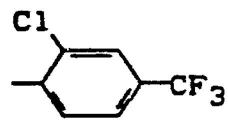 |
| $NO_2$ | H | - | - | 0 | 0 | 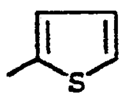 | 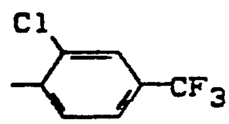 |
| H | H | - | - | 0 | 0 | 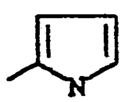 | 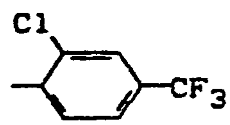 |
| $NO_2$ | H | - | - | 0 | 0 | 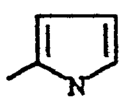 | 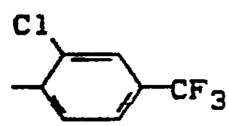 |
| H | H | - | - | 0 | 0 | 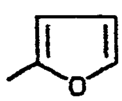 | 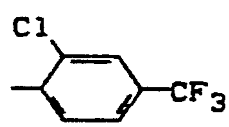 |

Tabelle 1 (Fortsetzung)

| $R^1$ | $R^2$ | $R^3$ | $R^4$ | n | X | Het | Ar |
|---|---|---|---|---|---|---|---|
| $NO_2$ | H | - | - | 0 | O | 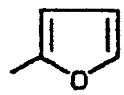 | 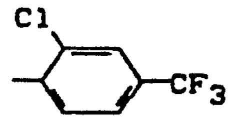 |
| H | H | - | - | 0 | O | 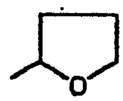 | 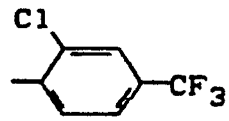 |
| $NO_2$ | H | - | - | 0 | O | 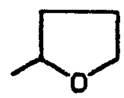 | 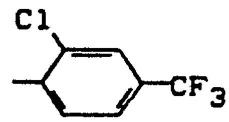 |
| H | $CH_3$ | - | - | 0 | O | 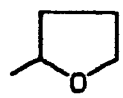 | 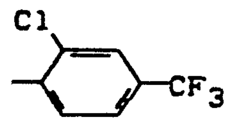 |
| $NO_2$ | $CH_3$ | - | - | 0 | O | 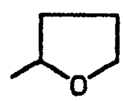 | 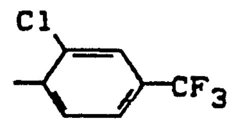 |
| H | H | - | - | 0 | S | 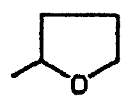 | 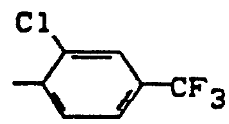 |
| $NO_2$ | H | - | - | 0 | S | 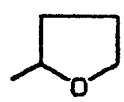 | 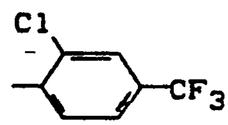 |
| H | H | - | - | 0 | O | 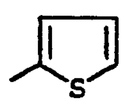 | 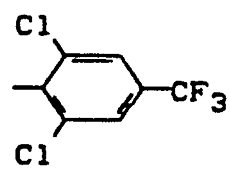 |

Tabelle 1 (Fortsetzung)

| $R^1$ | $R^2$ | $R^3$ | $R^4$ | n | X | Het | Ar |
|---|---|---|---|---|---|---|---|
| $NO_2$ | H | - | - | 0 | O | 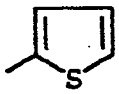 | 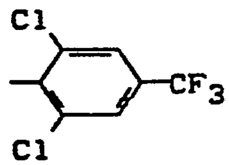 |
| H | H | - | - | 0 | O | 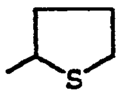 | 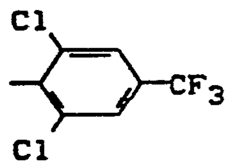 |
| $NO_2$ | H | - | - | 0 | O | 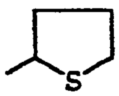 | 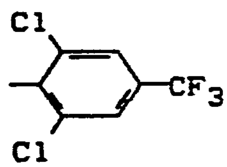 |
| H | H | - | - | 0 | S | 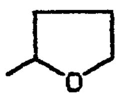 | 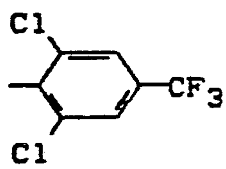 |
| $NO_2$ | H | - | - | 0 | S | 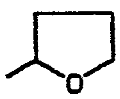 | 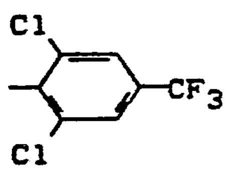 |
| H | H | - | - | 0 | O | 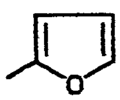 | 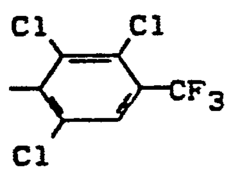 |
| $NO_2$ | H | - | - | 0 | O | 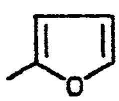 | 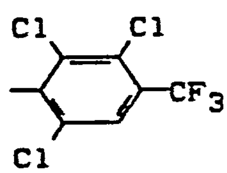 |

Tabelle 1 (Fortsetzung)

| $R^1$ | $R^2$ | $R^3$ | $R^4$ | n | X | Het | Ar |
|---|---|---|---|---|---|---|---|
| H | H | - | - | 0 | O | 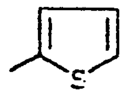 | 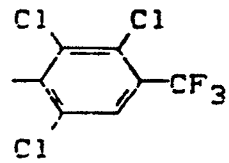 |
| $NO_2$ | H | - | - | 0 | O | 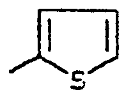 | 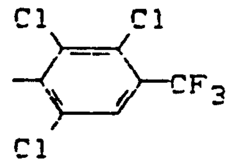 |
| H | H | - | - | 0 | O | 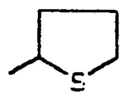 | 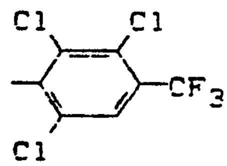 |
| $NO_2$ | H | - | - | 0 | O | 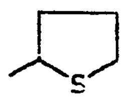 | 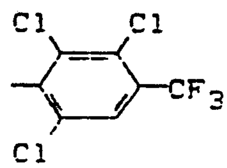 |
| H | H | - | - | 0 | O | 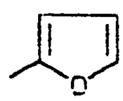 | 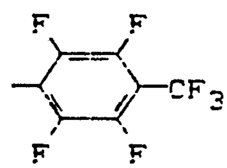 |
| $NO_2$ | H | - | - | 0 | O | 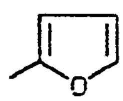 | 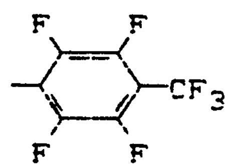 |
| H | H | - | - | 0 | O | 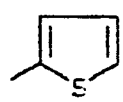 | 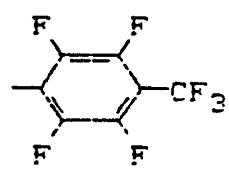 |
| $NO_2$ | H | - | - | 0 | O | 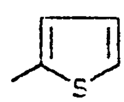 | 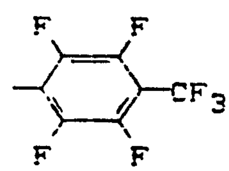 |

Tabelle 1 (Fortsetzung)

| $R^1$ | $R^2$ | $R^3$ | $R^4$ | n | X | Het | Ar |
|---|---|---|---|---|---|---|---|
| H | H | - | - | 0 | 0 | 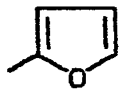 | 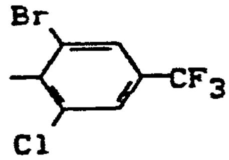 |
| $NO_2$ | H | - | - | 0 | 0 | 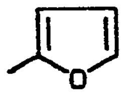 | 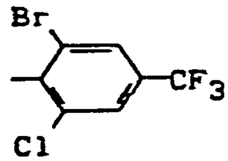 |
| H | H | - | - | 0 | 0 | 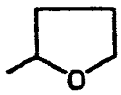 | 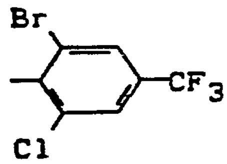 |
| $NO_2$ | H | - | - | 0 | 0 | 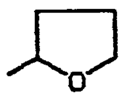 | 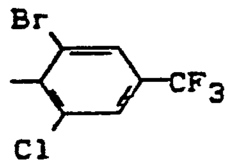 |
| H | H | - | - | 0 | 0 | 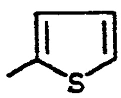 | 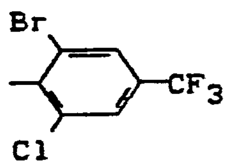 |
| $NO_2$ | H | - | - | 0 | 0 | 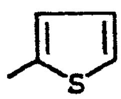 | 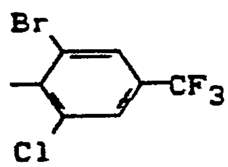 |
| H | H | - | - | 0 | 0 | 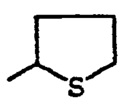 | 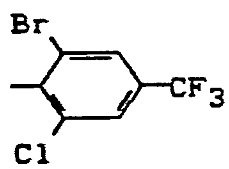 |
| $NO_2$ | H | - | - | 0 | 0 | 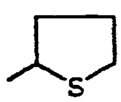 | 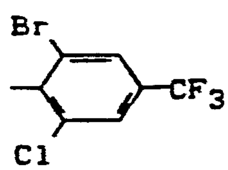 |

Tabelle 1 (Fortsetzung)

| $R^1$ | $R^2$ | $R^3$ | $R^4$ | n | X | Het | Ar |
|---|---|---|---|---|---|---|---|
| H | H | - | - | 0 | O | 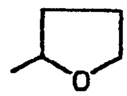 | 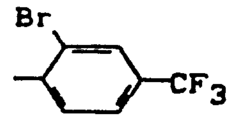 |
| $NO_2$ | H | - | - | 0 | O | 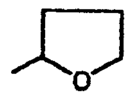 | 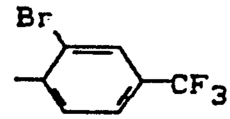 |
| H | H | - | - | 0 | O | 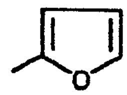 | 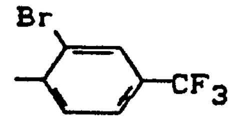 |
| $NO_2$ | H | - | - | 0 | O | 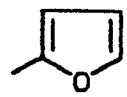 | 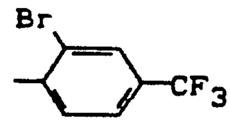 |
| $NO_2$ | H | - | - | 0 | O | 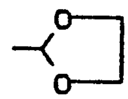 | 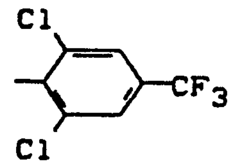 |
| $NO_2$ | H | - | - | 0 | O | 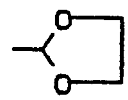 | 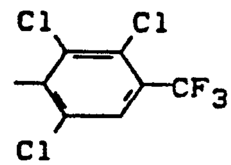 |
| $NO_2$ | H | - | - | 0 | O | 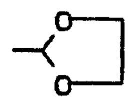 | 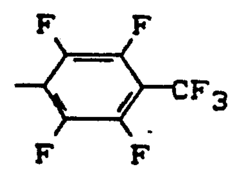 |
| $NO_2$ | H | - | - | 0 | O | 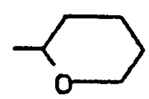 | 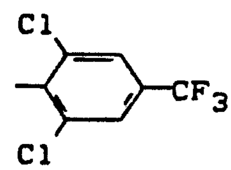 |
| $NO_2$ | H | - | - | 0 | O | 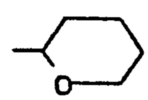 | 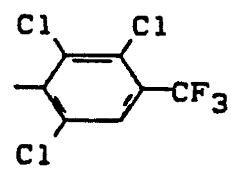 |

Tabelle 1 (Fortsetzung)

| $R^1$ | $R^2$ | $R^3$ | $R^4$ | n | X | Het | Ar |
|---|---|---|---|---|---|---|---|
| $NO_2$ | H | - | - | 0 | 0 | 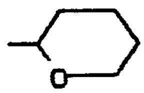 | 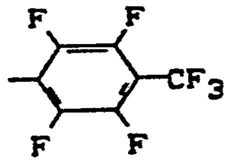 |
| $NO_2$ | H | - | - | 0 | 0 | 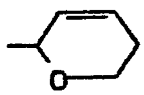 | 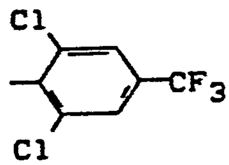 |
| $NO_2$ | H | - | - | 0 | 0 | 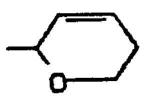 | 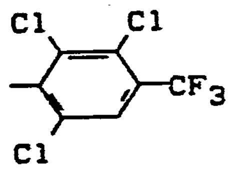 |
| $NO_2$ | H | - | - | 0 | 0 | 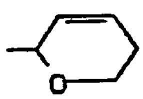 | 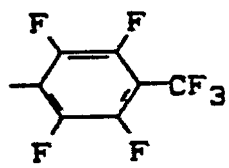 |
| $NO_2$ | H | - | - | 0 | 0 | 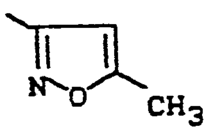 | 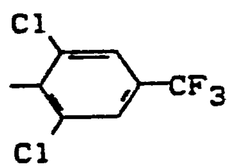 |
| $NO_2$ | H | - | - | 0 | 0 | 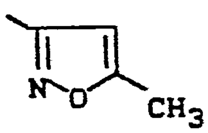 | 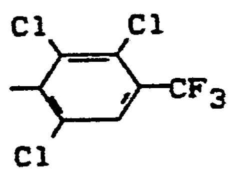 |
| $NO_2$ | H | - | - | 0 | 0 | 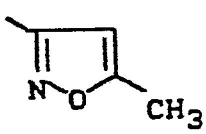 | 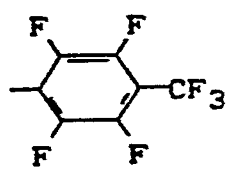 |
| $NO_2$ | H | - | - | 0 | 0 | 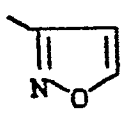 | 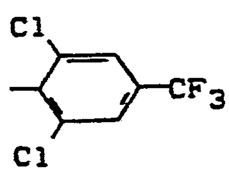 |

Tabelle 1 (Fortsetzung)

| $R^1$ | $R^2$ | $R^3$ | $R^4$ | n | X | Het | Ar |
|---|---|---|---|---|---|---|---|
| $NO_2$ | H | - | - | 0 | O | 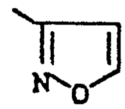 | 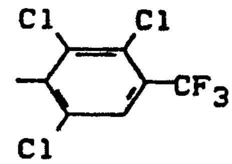 |
| $NO_2$ | H | - | - | 0 | O | 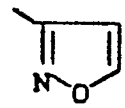 | 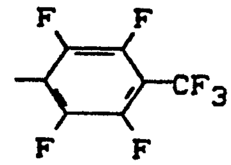 |
| $NO_2$ | H | - | - | 0 | O | 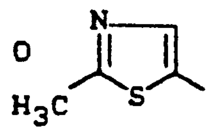 | 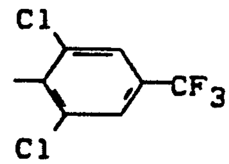 |
| $NO_2$ | H | - | - | 0 | O | 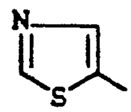 | 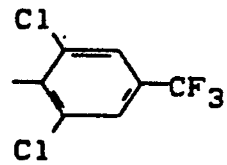 |
| $NO_2$ | H | - | - | 0 | O | 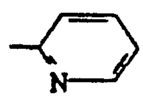 | 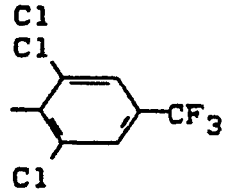 |
| $NO_2$ | H | - | - | 0 | O | 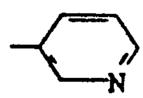 | 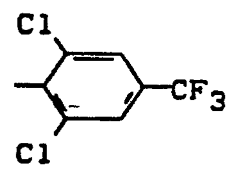 |
| $NO_2$ | H | - | - | 0 | O | 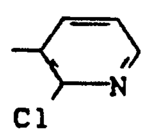 | 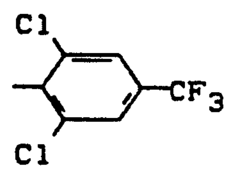 |
| $NO_2$ | H | - | - | 0 | O | 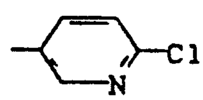 | 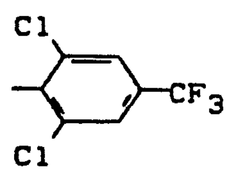 |

Tabelle 1 (Fortsetzung)

| $R^1$ | $R^2$ | $R^3$ | $R^4$ | n | X | Het | Ar |
|---|---|---|---|---|---|---|---|
| $NO_2$ | H | - | - | 0 | O | 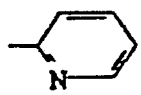 | 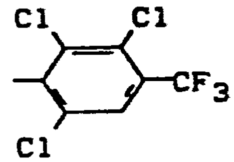 |
| $NO_2$ | H | - | - | 0 | O | 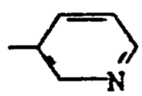 | 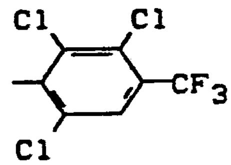 |
| $NO_2$ | H | - | - | 0 | O | 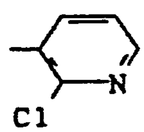 | 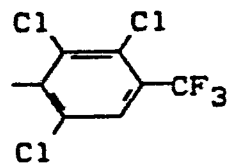 |
| $NO_2$ | H | - | - | 0 | O | 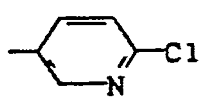 | 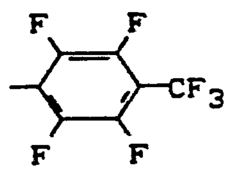 |
| $NO_2$ | H | - | - | 0 | O | 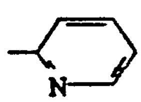 | 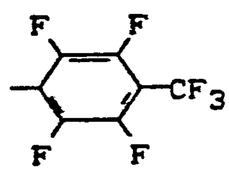 |
| $NO_2$ | H | - | - | 0 | O | 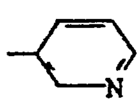 | 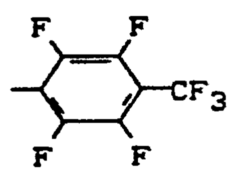 |

Tabelle 1 (Fortsetzung)

| $R^1$ | $R^2$ | $R^3$ | $R^4$ | n | X | Het | Ar |
|---|---|---|---|---|---|---|---|
| $NO_2$ | H | - | - | 0 | O | 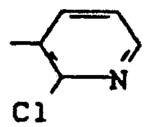 | 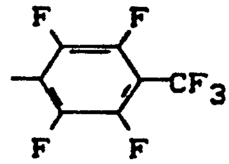 |
| $NO_2$ | H | - | - | 0 | O | 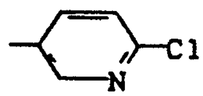 | 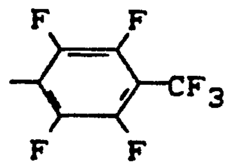 |

Verwendet man beispielsweise 5-Amino-4-chlor-1-(2,6-dichlor-4-trifluormethyl-phenyl)-pyrazol und Furan-2-carbonsäurechlorid als Ausgangsstoffe, so läßt sich der Reaktionsablauf des erfindungsgemäßen Verfahrens (a) durch das folgende Formelschema darstellen:

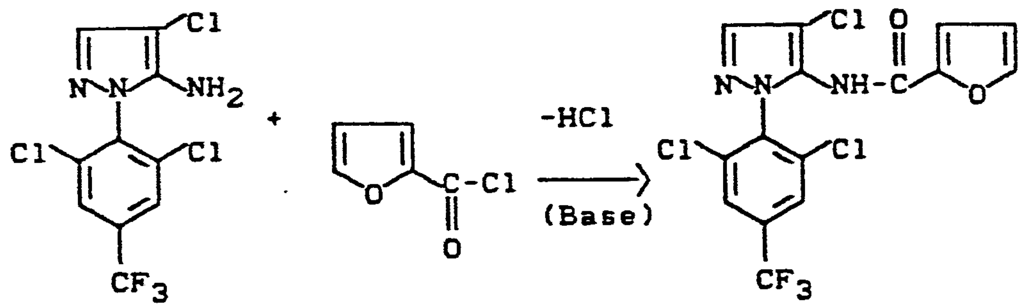


Verwendet man beispielsweise 5-(Furan-2-carbonamido)-1-(2,6-dichlor-4-trifluormethyl-phenyl)-pyrazol und Dimethylsulfat als Ausgangsstoffe, so läßt sich der Reaktionsablauf des erfindungsgemäßen Verfahrens (b) durch das folgende Formelschema darstellen:

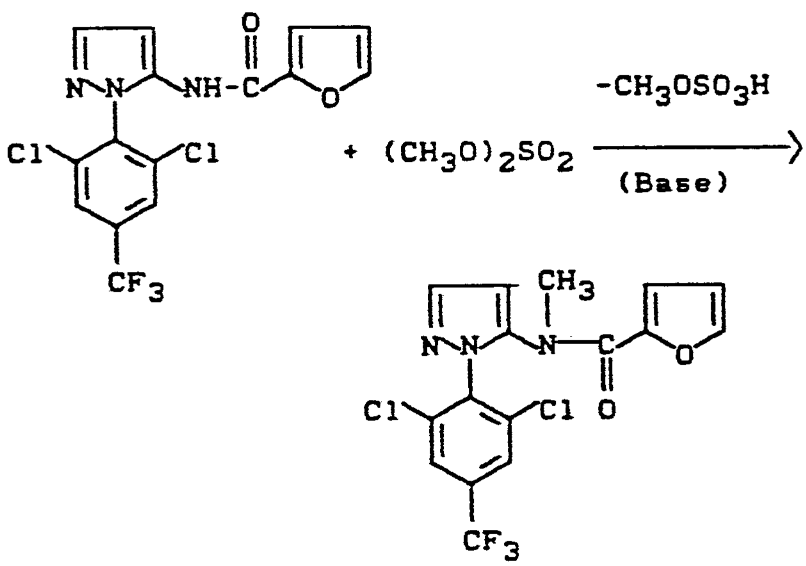

Verwendet man beispielsweise 5-(Tetrahydrofuran-2-carbonamido)-1-(2-chlor-4-trifluormethyl-phenyl)-pyrazol und Salpetersäure als Ausgangsstoffe, so läßt sich der Reaktionsablauf des erfindungsgemäßen Verfahrens (c) durch das folgende Formelschema darstellen:

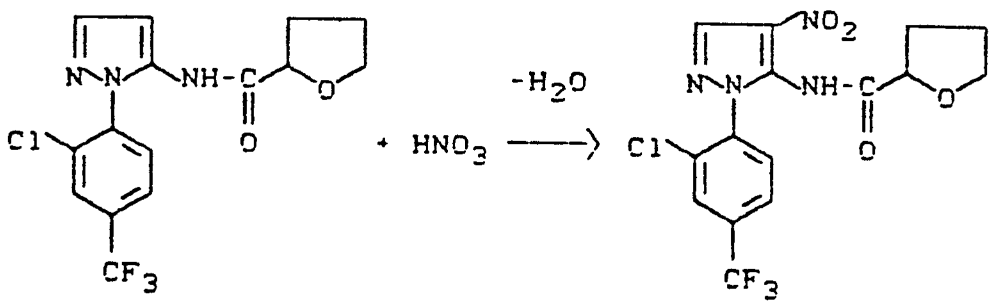


Die zur Durchführung des erfindungsgemäßen Verfahrens (a) als Ausgangsstoffe benötigten 5-Amino-1-aryl-pyrazole sind durch die Formel (II) allgemein definiert. In dieser Formel (II) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (II) sind teilweise bekannt (vgl. z.B. DE-OS 3 402 308), teilweise sind sie Gegenstand der eigenen vorgängigen noch nicht publizierten Patentanmeldung DE-P 3 520 330 vom 07.06.1985 und erhältlich in Analogie zu bekannten Verfahren (vgl. DE-OS 3 402 308), beispielsweise wenn man Arylhydrazine der Formel (VI),

$Ar - NH - NH_2$ (VI)

in welcher

Ar die oben angegebene Bedeutung hat,

mit 2-Halogenacrylnitrilen der Formel (VII),

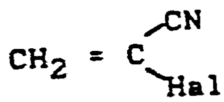 (VII)

in welcher

Hal für Halogen, insbesondere für Chlor oder Brom steht,

entweder zunächst in einer 1. Stufe, gegebenenfalls in Gegenwart eines Verdünnungsmittels wie beispielsweise Eisessig oder Ethanol sowie gegebenenfalls in Gegenwart eines Reaktionshilfsmittels wie beispielsweise Natriumacetat bei Temperaturen zwischen -20 °C und +20 °C umsetzt zu den Arylhydrazin-Derivaten der Formel (VIII)

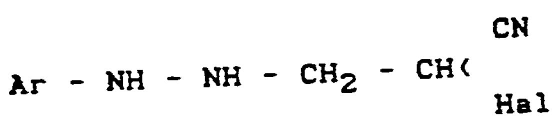 (VIII)

in welcher

Ar und Hal die oben angegebene Bedeutung haben,

und diese in einer 2. Stufe. gegebenenfalls in Gegenwart eines Verdünnungsmittels wie beispielsweise Ethylenglykolmonoethylether und gegebenenfalls in Gegenwart eines sauren Katalysators wie beispielweise Schwefelsäure oder Phosphorsäure bei Temperaturen zwischen +50 °C und +150 °C cyclisiert,

oder direkt in einem Reaktionsschritt ohne Isolierung der Zwischenstufe der Formel (VIII), gegebenenfalls in Gegenwart eines Verdünnungsmittels wie beispielsweise Ethylenglykolmonoethylether oder Ethanol bei Temperaturen zwischen +50 °C und +150 °C cyclisiert und die so erhältlichen 4-unsubstituierten 5-Amino-pyrazole der Formel (IIa),

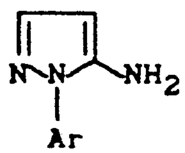

(IIa)

in welcher

Ar die oben angegebene Bedeutung hat,

in einer Folgereaktion mit einem Nitrierungsmittel, wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen -20 °C und +50 °C nitriert oder alternativ mit einem Halogenierungsmittel, wie beispielsweise Chlor, Sulfurylchlorid, Phosphorpentachlorid, N-Chlorsuccinimid, Brom, Phosphortribromid oder N-Bromsuccinimid, gegebenenfalls in Gegenwart eines Verdünnungsmittels, wie beispielsweise Methylenchlorid oder Eisessig, und gegebenenfalls in Gegenwart eines Reaktionshilfsmittels wie beispielsweise Bortrifluorid bei Temperaturen zwischen -20 °C und +50 °C halogeniert.
Dabei kann es gegebenenfalls von Vorteil sein, vor der Halogenierungs- bzw. Nitrierungsreaktion die Aminogruppe in der 5-Position des Pyrazolringes mit Hilfe üblicher Schutzgruppentechnik beispielsweise durch Acylierung zu schützen und die Aminoschutzgruppe nach erfolgter Halogenierung bzw. Nitrierung ebenfalls in üblicher Art und Weise beispielsweise durch Verseifung mit wässriger oder alkoholischer Base wieder abzuspalten.

Die Arylhydrazine (VI) sind bekannt (vgl. z.B. US-PS 4.127.575; US-PS 3.609.158; DE-OS 2 558 399;J. Chem. Soc. C. 1971, 167-174) oder können nach bekannten Verfahren in einfacher analoger Weise erhalten werden (vgl. z.B. Houben-Weyl "Methoden der organischen Chemie" Band X/2, S. 203, Thieme Verlag Stuttgart 1967; DE-OS 3 402 308).

Die Halogenacrylnitrile der Formel (VII) sind allgemein bekannte Verbindungen der organischen Chemie.

Die zur Durchführung des erfindungsgemäßen Verfahrens (a) weiterhin als Ausgangsstoffe benötigten Acylierungsmittel sind durch die Formel (III) allgemein definiert. In dieser Formel (III) stehen X, $R^3$, $R^4$, Het und der Index n vorzugsweise für diejenigen Reste, die bereits im Zusammenhang mit der Beschreibung der erfindungsgemäßen Stoffe der Formel (I) als bevorzugt für diese Substituenten genannt wurden.

$E^1$ steht vorzugsweise für Halogen, insbesondere Chlor oder Brom, oder für einen Rest 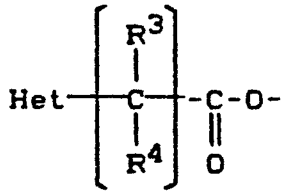, wobei X, $R^3$, $R^4$, Het und der Index n die oben angegebene Bedeutung haben. Die Acylierungsmittel der Formel (III) sind allgemein bekannte Verbindungen der organischen Chemie.

Die zur Durchführung des erfindungsgemäßen Verfahrens (b) als Ausgangsstoffe benötigten 5-Acylamino-pyrazol-Derivate sind durch die Formel (Ia) allgemein definiert. In dieser Formel (Ia) stehen $R^1$, X, $R^3$, $R^4$, Ar, Het und der Index n vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Acylamino-pyrazol-Derivate der Formel (Ia) sind erfindungsgemäße Verbindungen und erhältlich mit Hilfe der erfindungsgemäßen Verfahren (a) oder (c).

Die zur Durchführung des erfindunsggemäßen Verfahrens (b) weiterhin als Ausgangsstoffe benötigten Alkylierungsmittel sind durch die Formel (IV) allgemein definiert. In dieser Formel (IV) steht $R^{2-1}$ vorzugsweise für geradkettiges oder verzweigtes Alkyl mit 1 bis 12 Kohlenstoffatomen, für geradkettiges oder verzweigtes Alkenyl und Alkinyl mit jeweils 3 bis 8 Kohlenstoffatomen, sowie für gegebenenfalls einfach

bis zweifach, gleich oder verschieden substituiertes Cycloalkyl mit 3 bis 8 Kohlenstoffatomen, wobei als Substituenten Halogen und Alkyl mit 1 bis 4 Kohlenstoffatomen genannt seien. $E^2$ steht vorzugsweise für Chlor, Brom oder Iod, sowie für Methoxysulfonyloxy oder für p-Toluolsulfonyloxy.

Die Alkylierungsmittel der Formel (IV) sind allgemein bekannte Verbindungen der organischen Chemie.

Die zur Durchführung des erfindungsgemäßen Verfahrens (c) als Ausgangsstoffe benötigten 5-Acylamino-pyrazol-Derivate sind durch die Formel (Id) allgemein definiert. In dieser Formel (Id) stehen $R^2$, X, $R^3$, $R^4$, Ar, Het und der Index n vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Acylamino-pyrazol-Derivate der Formel (Id) sind erfindungsgemäße Verbindungen und erhältlich mit Hilfe der erfindungsgemäßen Verfahren (a) oder (b).

Die zur Durchführung des erfindungsgemäßen Verfahrens (c) weiterhin als Ausgangsstoffe benötigten Halogenierungs- oder Nitrierungsmittel sind durch die Formel (V) allgemein definiert. In dieser Formel (V) steht $R^{1-1}$ vorzugsweise für Chlor, Brom oder Nitro.
$E^3$ steht vorzugsweise für eine übliche Abgangsgruppe, wie beispielsweise Halogen sowie phosphor- ooder schwefelhaltige halogenierte Abgangsgruppen. Geeignete Halogenierungs- und Nitrierungsmittel sind beispielsweise elementares Chlor oder Brom, Salpetersäure, Nitriersäure, Sulfurylchlorid,

Phosphoroxychlorid, Phosphoroxybromid, Phosphortribromid und ähnliche allgemein übliche Halogenierungs- und Nitrierungsmittel.

Die Halogenierungs- und Nitrierungsmittel der Formel (V) sind allgemein bekannte Verbindungen.

Als Verdünnungsmittel zur Durchführung des erfindungsgemäßen Verfahrens (a) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxdid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Das erfindungsgemäßen Verfahren (a) kann auch gegebenenfalls in Gegenwart eines geeigneten Acylierungskatalysators durchgeführt werden. Als solche verwendet man vorzugsweise Protonensäuren wie Schwefelsäure, Salzsäure, Phosphorsäure, Trifluoressigsäure oder Lewis-Säuren wie Aluminiumtrichlorid, Bortrifluorid oder Eisentrichlorid. Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und +150 °C, vorzugsweise bei Temperaturen zwischen 0 °C und 100 °C.

Zur Durchführung des erfindungsgemäßen Verfahrens (a) setzt man pro Mol an 5-Amino-1-aryl-pyrazol der Formel (II) im allgemeinen 1.0 bis 15.0 Mol, vorzugsweise 1.0 bis 5.0 Mol an Acylierungsmittel der Formel (III), gegebenenfalls 1.0 bis 3.0 Mol, vorzugsweise 1.0 bis 2.0 Mol an Säurebindemittel oder gegebenenfalls 0.1 bis 3.0 Mol, vorzugsweise 0,1 bis 2.0 Mol an Acylierungskatalysator

ein. Die Reaktionsdurchführung, Aufarbeitung und Isolierung der Reaktionsprodukte der Formel (Ia) erfolgt nach üblichen, bekannten Methoden.

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten organischen Lösungsmittel.

Das erfindungsgemäße Verfahren (b) kann gegebenenfalls auch in einem Zweiphasensystem, wie beispielsweise Wasser/Toluol oder Wasser/Dichlormethan, und gegebenenfalls in Gegenwart eines Phasentransferkatalysators durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dimethyl-dibenzyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.
Als Säurebindemittel zur Durchführung des Herstellungsverfahrens (b) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +150 °C, vorzugsweise bei Temperaturen zwischen 0 °C und +100 °C.

Zur Durchführung des erfindungsgemäßen Verfahrens (b) setzt man pro Mol an 5-Acylamino-pyrazol-Derivat der Formel (Ia) im allgemeinen 1.0 bis 20.0 Mol, vorzugsweise 1.0 bis 15.0 Mol an Alkylierungsmittel der Formel (IV) und gegebenenfalls 1.0 bis 3.0 Mol, vorzugsweise 1.0 bis 2.0 Mol an Säurebindemittel sowie gegebenenfalls 0.01 bis 1.0 Mol an Phasentransferkatalysator ein. Die Reaktionsdurchführung, Aufarbeitung und Isolierung der Reaktionsprodukte der Formel (Ib) erfolgt in allgemein üblicher Art und Weise.

Als Verdünnungsmittel zur Durchführung des Herstellungsverfahrens (c) kommen alle üblicherweise für derartige elektrophile Substitutionen verwendbaren Lösungsmittel infrage. Vorzugsweise verwendet man die als Reagenzien infrage kommenden Säuren oder Gemische, wie beispielsweise Salpetersäure, Nitriersäure oder Sulfurylchlorid gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel infrage.

Als Katalysatoren oder Reaktionshilfsmittel zur Durchführung des Herstellungsverfahrens (c) kommen ebenfalls die für derartige Reaktionen üblichen Katalysatoren infrage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (c) in einem größeren Bereich variiert werden. Im allgemeinen arbeitet man zwischen -50 °C und +200 °C, vorzugsweise zwischen -20 °C und +150 °C.

Zur Durchführung des Herstellungsverfahrens (c) setzt man pro Mol 5-Acylamino-pyrazol-Derivat der Formel (Id) im allgemeinen 1.0 bis 10.0 Mol, vorzugsweise 1.0 bis 5.0 Mol an Halogenierungs- oder Nitrierungsmittel der Formel (V) und gegebenenfalls 0.1 bis 10 Mol vorzugsweise 1,0 bis 5,0 Mol an Katalysator oder Reaktionshilfsmittel ein. Die Reaktionsführung, Aufarbeitung und Isolierung der Reaktionsprodukte der Formel (Ic) erfolgt in allgemein üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von mono- und dikotylen Unkräutern in mono- und dikotylen Kulturen, wie beispielsweise Soja, Gerste, Weizen oder Mais einsetzen.

Auch die Zwischenprodukte der Formel (II) besitzen eine gute herbizide Wirksamkeit.

Die erfindungsgemäßen Wirkstoffe greifen auch in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie

Butan, Propan, Stickstoff und Kohlendioxid.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B., 4-Amino-6-t-butyl-3-ethylthio-1,2,4-triazin-5(4H)-on oder N-(2-Benzthiazolyl)-N,N'-dimethyl-harnstoff zur Unkrautbekämpfung im Getreide;4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-chlor-4-methylphenyl)-harnstoff; N,N-Dimethyl-N'-(4-isopropylphenyl)-harnstoff; 1-Amino-6-ethylthio-3-(2,2-dimethylpropyl)-1,3,5-triazin-2,4(1H,3H)-dion 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-[4-(3,5-Dichlorpyrid-2-yloxy)-phenoxy]-propionsäure-(2-benzyloxy-ethylester);-(trimethylsilylmethylester) oder -(2,2-diethoxyethylester); 3,5-Diiod-4-hydroxybenzonitril); 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; N-(1-Ethylpropyl)-3,4-dimethyl-2,6-dinitroanilin; O-(6-chlor-3-phenyl-pyridazin-4-yl)-S-octyl-thiocarbonat; N,N-Diisopropyl-S-(2,3,3-trichlorallyl)-thiolcarbamat; N-Methyl-2-(1,3-benzthiazol-2-yloxy)-acetanilid; exo-1-Methyl-4-(1-methylethyl)-2-(2-methylphenyl-methoxy)-7-oxabicyclo-(2,2,1)-heptan; 2-{-4-[(3-Chlor-5-(trifluormethyl)-2-pyridinyl)-oxy]-phenoxy}-propionsäure bzw. -propansäureethylester;3,5-Dibrom-4-hydroxy-benzonitril; 2-[5-Methyl-5-(1-methylethyl)-4-oxo-2-imidazolin-2-yl]-3-chinolincarbonsäure oder 1-(3-Trifluormethyl-phenyl)-4-methylamino-5-chlor-pyridazon-(6) sind gegebenenfalls von Vorteil.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Aufwandmengen können bei der Verwendung der Wirkstoffe als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele:

Beispiel 1

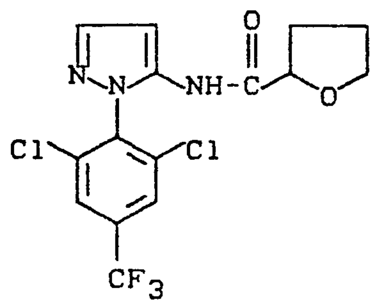

(Verfahren a)

19,8g (0,07 Mol) 5-Amino-1-(2,6-dichlor-4-trifluormethyl-phenyl)-pyrazol und 7,1g (0,09 Mol) Pyridin werden in 150ml Acetonitril gelöst und unter Rühren bei 25-30° C mit 12,1g (0,09 Mol) Tetrahydrofuran-2-carbonsäurechlorid versetzt. Es wird noch 24 Stunden bei Raumtemperatur gerührt. Anschließend wird der Reaktionsansatz auf Wasser gegossen und mit Chloroform extrahiert; die Chloroformphase mit wässriger Natriumhydrogencarbonat-Lösung gewaschen, über Natriumsulfat getrocknet und eingeengt. Der ölige Rückstand wird durch Anreiben mit Petrolether kristallisiert.

Man erhält 24 g (87 % d.Theorie) 1-(2,6-Dichlor-4-trifluormethylphenyl)-5-(2-tetrahydrofuroyl)-amino-pyrazol vom Schmelzpunkt 95-97° C.

Herstellung des Ausgangsproduktes

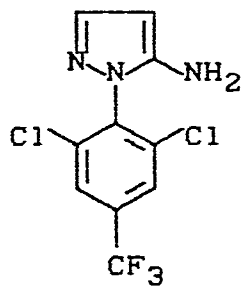

24,5g (0,1 Mol) 2,6-Dichlor-4-trifluormethylphenylhydrazin und 20 mg Ethylendiamin-tetraessigsäure-Dinatriumsalz (Titriplex III) in 150 ml Methanol werden bei Rückflußtemperatur tropfenweise mit 25 ml (27,6g/0,3 Mol) 2-Chloracrylnitril versetzt. Nach beendeter Zugabe erhitzt man weitere 8 Stunden auf Rückflußtemperatur, tropft 9 ml (0,16 Mol) 96 %ige Schwefelsäure zu und erhitzt weitere 6 Stunden auf Rückflußtemperatur. Die abgekühlte Reaktionsmischung wird mit 33,5g (0,3 Mol) wasserfreiem Natriumcarbonat versetzt. Nach 4 Stunden entfernt man das Lösungsmittel im Vakuum, nimmt den Rückstand in 500 ml Wasser auf und rührt 10 Stunden bei Raumtemperatur. Man filtriert den ausgefallenen Niederschlag ab, wäscht mit Wasser nach und trocknet im Vakuum bei 50° C.
Man erhält 28,5g (96 % der Theorie) 5-Amino-1-(2,6-dichlor-4-trifluormethylphenyl)-pyrazol vom Schmelzpunkt 103-105° C.

Beispiel 2

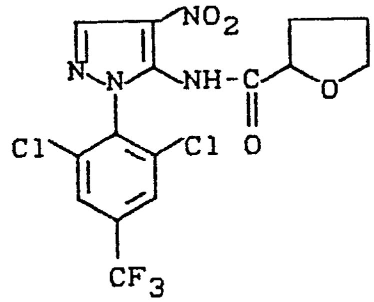

(Verfahren c)

10,6g (0,027 Mol) 1-(2,6-Dichlor-4-trifluormethylphenyl)-5-(2-tetrahydrofuroyl)-amino-pyrazol (Bsp.1) und 3 ml Acetanhydrid werden in 50 ml Eisessig gelöst und bei 10°C unter Kühlung mit 2,2 ml konzentrierter Salpetersäure versetzt. Anschließend wird noch 24 Stunden bei Raumtemperatur gerührt, in Wasser eingegossen, mit Methylenchlorid extrahiert, die Methylenchlorid-Phase anschließend noch mit wässriger Natriumhydrogencarbonatlösung gewaschen, über Natriumsulfat getrocknet und eingeengt.

Man erhält 11g (93 % der Theorie) 1-(2,6-Dichlorphenyl-4-trifluormethylphenyl)-4-nitro-5-(2-tetrahydrofuroyl)-amino-pyrazol vom Fp.: 169-172°C.

Beispiel 3

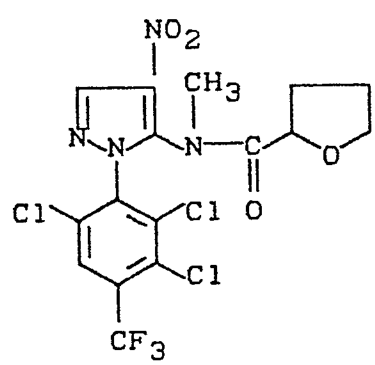

(Verfahren b)

5,2 g (0,011 Mol) 1-(2,3,6-Trichlor-4-trifluormethyl-phenyl)-4-nitro-5-(2-tetrahydrofuroyl)-amino-pyrazol werden in 40 ml Acetonitril gelöst und nach Zugabe von 1,8 g (0,0132 Mol) gepulvertem Kaliumcarbonat bei ca. 50°C tropfenweise mit 1,7 g (0,0132 Mol) Dimethylsulfat versetzt. Es wird noch ca. 8 Stunden bei 50 bis 60°C nachgerührt, die Reaktionsmischung anschließend filtriert und im Vakuum vom Lösungsmittel befreit. Der Rückstand wird mit Methylenchlorid aufgenommen, mehrmals mit Wasser gewaschen, die organische Phase sodann über Natriumsulfat getrocknet und eingeengt. Es verbleiben 5 g eines Öls, das über Kieselgel chromatografiert wird (Laufmittel: Petrolether-Essigester 8:2). Die Hauptfraktion kristallisiert durch verreiben mit Diisopropylether.

Man erhält 2,2 g (41 % der Theorie) 1-(2,3,6-Trichlor-4-trifluormethyl-phenyl)-4-nitro-5-(N-methyl-N-tetrahydrofuro-2-yl-)amino-pyrazol vom Schmelzpunkt 142 bis 143°C.

Gemäß den Herstellungsbeispielen und den allgemeinen Angaben zur Herstellung werden die in der folgenden Tabelle 2 aufgeführten Verbindungen der Formel (I) erhalten

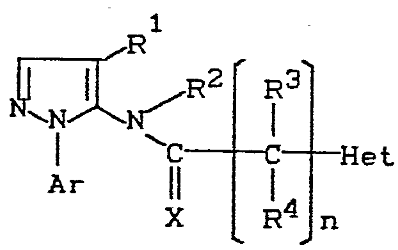

(I)

Tabelle 2

| Bsp. Nr. | $R^1$ | $R^2$ | n | $R^3$ | $R^4$ | X | Het | Ar | Fp(°C) |
|---|---|---|---|---|---|---|---|---|---|
| 4 | H | H | 0 | - | - | O | 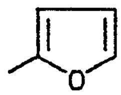 | 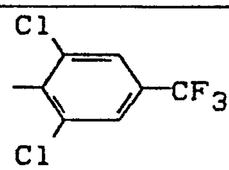 | 157-162 |
| 5 | $NO_2$ | H | 0 | - | - | O | 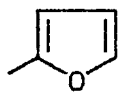 | 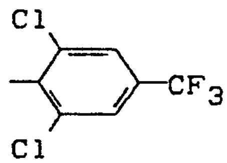 | 154-158 |
| 6 | H | H | 0 | - | - | O | 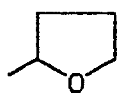 | 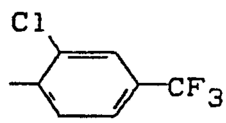 | 67-71 |
| 7 | $NO_2$ | H | 0 | - | - | O | 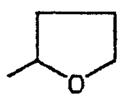 | 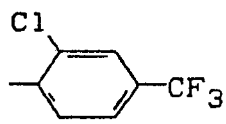 | 98-102 |
| 8 | H | H | 0 | - | - | O | 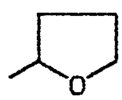 | 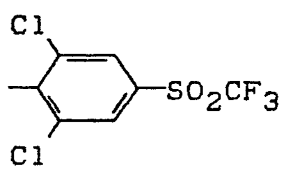 | 145-151 |
| 9 | $NO_2$ | H | 0 | - | - | O | 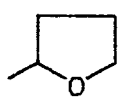 | 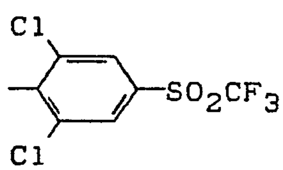 | 138-140 |

Tabelle 2 (Fortsetzung)

| Bsp. Nr. | $R^1$ | $R^2$ | n | $R^3$ | $R^4$ | X | Het | Ar | Fp(°C) |
|---|---|---|---|---|---|---|---|---|---|
| 10 | H | H | 0 | - | - | O | 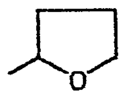 | 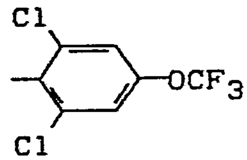 | 67-71 |
| 11 | $NO_2$ | H | 0 | - | - | O | 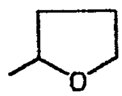 | 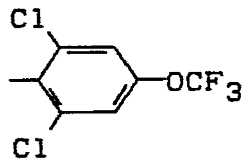 | 129-131 |
| 12 | H | H | 0 | - | - | O | 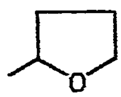 | 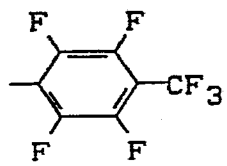 | Öl |
| 13 | $NO_2$ | H | 0 | - | - | O | 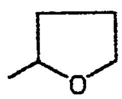 | 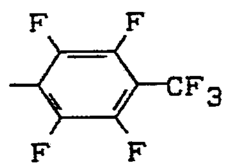 | 69-71 |
| 14 | H | H | 0 | - | - | O | 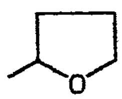 | 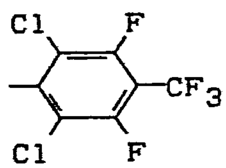 | Öl |
| 15 | $NO_2$ | H | 0 | - | - | O | 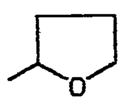 | 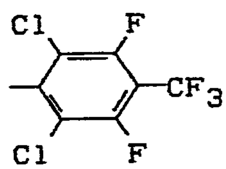 | 119-121 |

Tabelle 2 (Fortsetzung)

| Bsp. Nr. | $R^1$ | $R^2$ | n | $R^3$ | $R^4$ | X | Het | Ar | Fp(°C) |
|---|---|---|---|---|---|---|---|---|---|
| 16 | H | H | 0 | - | - | O | 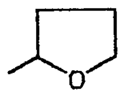 | 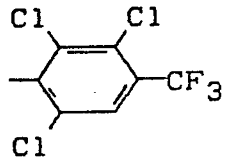 | Öl |
| 17 | $NO_2$ | H | 0 | - | - | O | 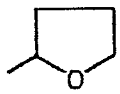 | 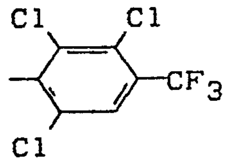 | 129 |
| 18 | H | H | 0 | - | - | O | 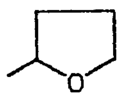 | 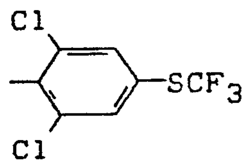 | 90 |
| 19 | $NO_2$ | H | 0 | - | - | O | 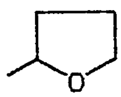 | 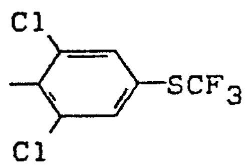 | 95 |
| 20 | H | H | 0 | - | - | O | 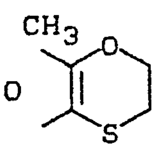 | 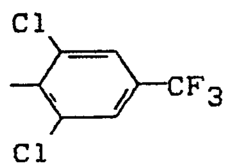 | 148-56 |
| 21 | $NO_2$ | H | 0 | - | - | O | 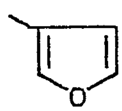 | 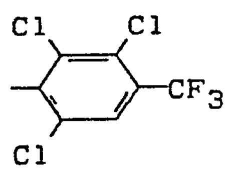 | |

Tabelle 2 (Fortsetzung)

| Bsp. Nr. | $R^1$ | $R^2$ | n | $R^3$ | $R^4$ | X | Het | Ar | Fp(°C) |
|---|---|---|---|---|---|---|---|---|---|
| 22 | H | H | 0 | - | - | O | 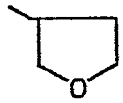 | 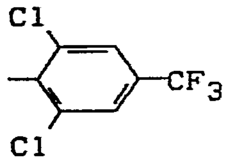 | 183-85 |
| 23 | H | H | 0 | - | - | O | 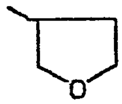 | 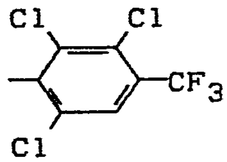 | 170-73 |
| 24 | H | H | 0 | - | - | O | 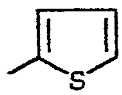 | 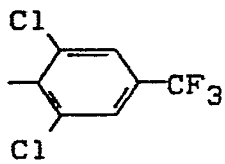 | 184-86 |
| 25 | H | H | 0 | - | - | O | 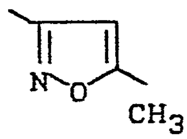 | 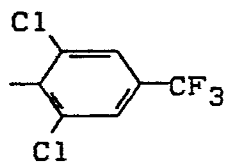 | 145-48 |
| 26 | H | H | 0 | - | - | O | 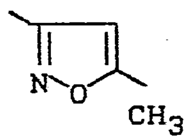 | 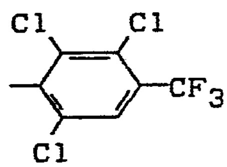 | 85-86 |
| 27 | H | H | 0 | - | - | O | 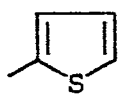 | 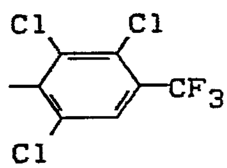 | 186-89 |

Tabelle 2 (Fortsetzung)

| Bsp. Nr. | $R^1$ | $R^2$ | n | $R^3$ | $R^4$ | X | Het | Ar | Fp(°C) |
|---|---|---|---|---|---|---|---|---|---|
| 28 | $NO_2$ | H | 0 | - | - | O | 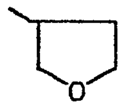 | 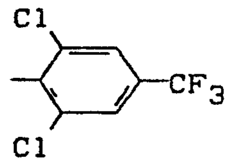 | 168-71 |
| 29 | $NO_2$ | H | 0 | - | - | O | 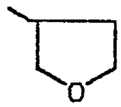 | 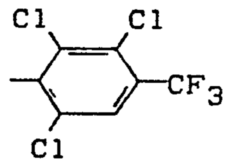 | 135-40 |
| 30 | $NO_2$ | H | 0 | - | - | O | 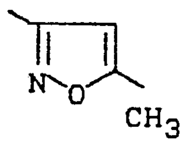 | 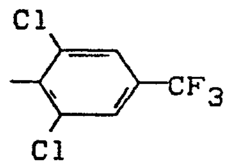 | 211-14 |
| 31 | H | H | 0 | - | - | O | 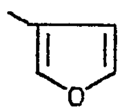 | 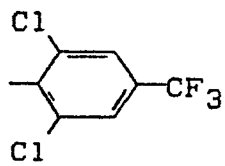 | 223-25 |
| 32 | H | H | 0 | - | - | O | 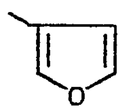 | 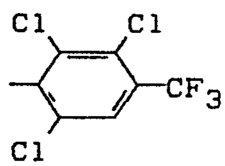 | 206-08 |
| 33 | $NO_2$ | H | 0 | - | - | O | 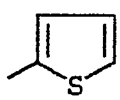 | 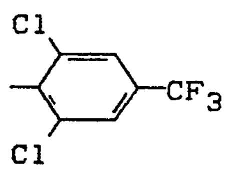 | 190-94 |

Tabelle 2 (Fortsetzung)

| Bsp. Nr. | $R^1$ | $R^2$ | n | $R^3$ | $R^4$ | X | Het | Ar | Fp(°C) |
|---|---|---|---|---|---|---|---|---|---|
| 34 | $NO_2$ | H | 0 | - | - | O | 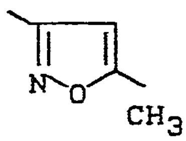 | 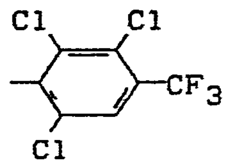 | 149-52 |
| 35 | $NO_2$ | H | 0 | - | - | O | 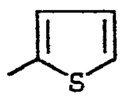 | 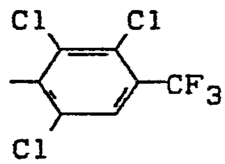 | 179-82 |
| 36 | $NO_2$ | H | 0 | - | - | O | 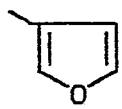 | 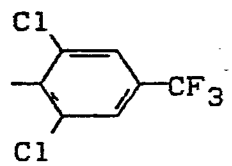 | |
| 37 | $NO_2$ | H | 0 | - | - | O | 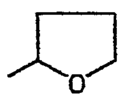 | 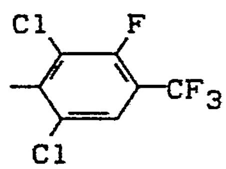 | 137 |
| 38 | $NO_2$ | H | 0 | - | - | S | 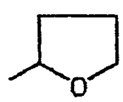 | 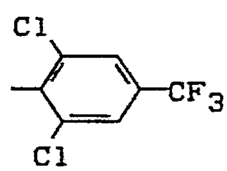 | 137 |
| 39 | H | H | 0 | - | - | O | 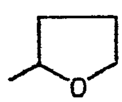 | 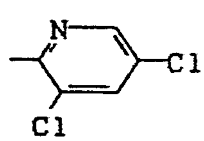 | 88 |
| 40 | $NO_2$ | H | 0 | - | - | O | 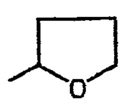 | 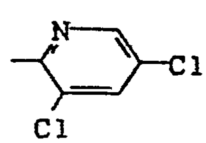 | 56 |

Anwendungsbeispiele

In den folgenden Anwendungsbeispielen wurde die nachstehend aufgeführte Verbindung als Vergleichssubstanz eingesetzt:

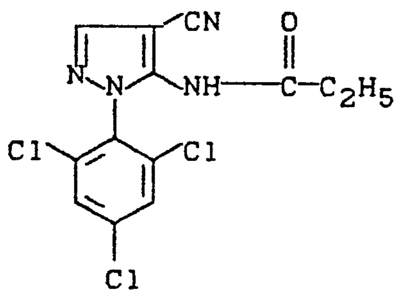

(A)

4-Cyano-5-propionamido-1-(2,4,6-trichlorphenyl)-pyrazol (bekannt aus DE-OS 32 26 513)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Kulturpflanzenselektivität gegenüber der Vergleichssubstanz (A) zeigen in diesem Test z.B. die Verbindungen gemäß den Herstellungsbeispielen 2, 5, 13, 15, 17 und 19.

## Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit ebenso wie in der Kulturpflanzenselektivität gegenüber der Vergleichssubstanz (A) zeigen in diesem Test z.B. die Verbindungen gemäß den Herstellungsbeispielen 2, 5, 7, 9, 11, 13, 15, 17 und 19.

## Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

In diesem Test zeigen z.B. die Wirkstoffe gemäß den Herstellungsbeispielen 2, 5, 7, 9, 13, 15, 17, 21, 28, 29, 34, 36 und 38 eine gute Wirkung.

Patentansprüche

1. 5-Acylamino-pyrazol-Derivate der allgemeinen Formel (I),

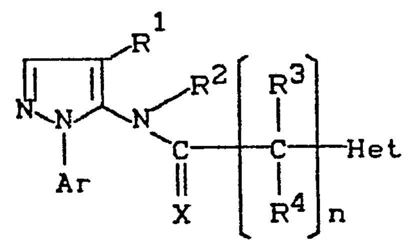

(I)

in welcher

$R^1$ für Wasserstoff, Halogen oder Nitro steht,

$R^2$ für Wasserstoff, Alkyl, Alkenyl, Alkinyl oder gegebenenfalls substituiertes Cycloalkyl steht,

$R^3$ für Wasserstoff oder Alkyl steht,

$R^4$ für Wasserstoff oder Alkyl steht,

X für Sauerstoff oder Schwefel steht,

n für die ganzen Zahlen 0, 1 oder 2 steht,

Ar für jeweils gegebenenfalls substituiertes Phenyl oder Pyridyl steht und

Het für einen gegebenenfalls substituierten 5- oder 6-gliedrigen, über ein Kohlenstoffatom verknüpften Heterocyclus steht.

2. 5-Acylamino-pyrazol-Derivate der Formel (I) gemäß Anspruch 1,

in welcher

$R^1$ für Wasserstoff, Nitro, Fluor, Chlor, Brom oder Iod steht;

$R^2$ für Wasserstoff, für geradkettiges oder verzweigtes Alkyl mit 1 bis 12 Kohlenstoffatomen, für geradkettiges oder verzweigtes Alkenyl und Alkinyl mit jeweils 3 bis 8 Kohlenstoffatomen, sowie für gegebenenfalls einfach oder zweifach, gleich oder verschieden substituiertes Cycloalkyl mit 3 bis 8 Kohlenstoffatomen steht, wobei als Substituenten Halogen und Alkyl mit 1 bis 4 Kohlenstoffatomen genannt seien;

$R^3$ für Wasserstoff oder für geradkettiges oder verzweigtes Alkyl mit 1 bis 6 Kohlenstoffatomen steht;

$R^4$ für Wasserstoff oder für geradkettiges oder verzweigtes Alkyl mit 1 bis 6 Kohlenstoffatomen steht;

X für Sauerstoff oder Schwefel steht;

n für die ganzen Zahlen 0, 1 oder 2 steht;

Ar für gegebenenfalls einfach oder mehrfach, gleich oder verschieden substituiertes Phenyl oder für jeweils gegebenenfalls einfach oder mehrfach, gleich oder verschieden substituiertes 2-Pyridyl, 3-Pyridyl oder 4-Pyridyl steht, wobei als Phenyl- und Pyridyl-Substituenten jeweils in Frage kommen: Cyano, Nitro, Halogen, jeweils geradkettiges oder verzweigtes Alkyl, Alkoxy oder Alkoxycarbonyl mit jeweils 1 bis 4 Kohlenstofftomen im Alkylteil, außerdem jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^5$,

wobei

$R^5$ für Amino sowie für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

m für die ganzen Zahlen 0, 1 oder 2 steht;

Het für einen gegebenenfalls einfach bis dreifach, gleich oder verschieden substituierten 5- oder 6-gliedrigen, über ein Kohlenstoffatom verknüpften, ungesättigten, teilweise gesättigten oder gesättigten Heterocyclus mit ein bis drei gleichen oder verschiedenen Heteroatomen steht, wobei als Substituenten genannt seien: Halogen, Alkyl, Alkoxy und Alkylthio mit jeweils 1 bis 4 Kohlenstoffatomen, sowie Halogenalkyl, Halogenalkoxy und Halogenalkylthio mit jeweils 1 bis 2 Kohlenstoffatomen und 1 bis 5 gleichen oder verschiedenen Halogenatomen.

3. 5-Acylamino-pyrazol-Derivate der Formel (I) gemäß Anspruch 1, in welcher,

$R^1$ für Wasserstoff, Nitro, Chlor oder Brom steht;

$R^2$ für Wasserstoff, für geradkettiges oder verzweigtes Alkyl mit 1 bis 8 Kohlenstoffatomen, sowie für geradkettiges oder verzweigtes Alkenyl und Alkinyl mit jeweils 3 bis 6 Kohlenstoffatomen steht;

$R^3$ für Wasserstoff oder Methyl steht;

$R^4$ für Wasserstoff oder Methyl steht;

X für Sauerstoff oder Schwefel steht;

n für die ganzen Zahlen 0 oder 1 steht;

Ar für gegebenenfalls ein- bis fünffach, gleich oder verschieden substituiertes Phenyl oder für jeweils gegebenenfalls ein-bis vierfach, gleich

oder verschieden substituiertes 2-Pyridyl, 3-Pyridyl oder 4-Pyridyl steht, wobei als Phenyl- und Pyridyl-Substituenten jeweils in Frage kommen: Cyano, Nitro, Fluor, Chlor, Brom, Iod, Methyl, Ethyl, n- und i-Propyl, n-, i-, s-und t-Butyl, Methoxy, Ethoxy, Methoxycarbonyl, Ethoxycarbonyl, Trifluormethyl, Trichlormethyl, Difluorchlormethyl, Difluorchlorethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^5$,

wobei

$R^5$ für Amino, Methylamino, Ethylamino, Dimethylamino, Diethylamino, Fluordichlormethyl, Difluormethyl, Tetrafluorethyl, Trifluorchlorethyl, Trichlorethyl, Trichlormethyl, Trifluormethyl, Methyl oder Ethyl steht,

und

m für die ganzen Zahlen 0, 1 oder 2 steht,

Het für einen gegebenenfalls einfach bis dreifach, gleich oder verschieden substituierten 5- oder 6-gliedrigen, über ein Kohlenstoffatom verknüpften, ungesättigten, teilweise gesättigten oder gesättigten Heterocyclus mit ein bis drei gleichen oder verschiedenen Heteroatomen (Sauerstoff, Stickstoff, Schwefel) steht, wobei als Substituenten genannt seien: Chlor, Brom, Methyl, Methoxy, Methylthio und Trifluormethyl.

4. 5-Acylamino-pyrazol-Derivate der Formel (I),

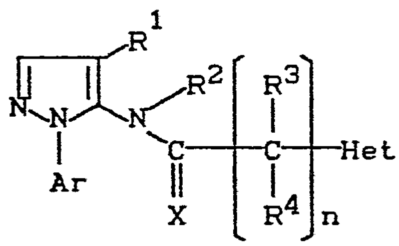

(I)

dadurch gekennzeichnet, daß $R^1$, $R^2$, $R^3$, $R^4$, Ar, X und n die in Anspruch 3 angegebene Bedeutung haben und Het für

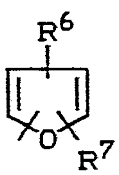
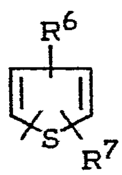
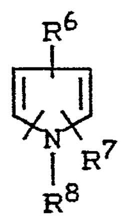
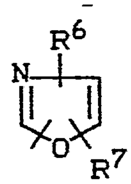
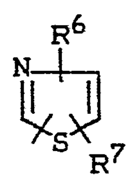

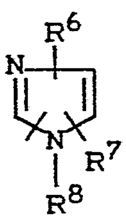
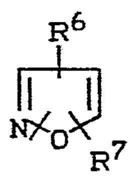
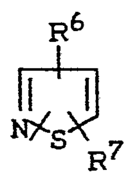
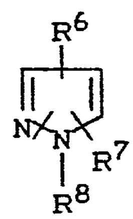
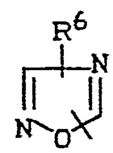

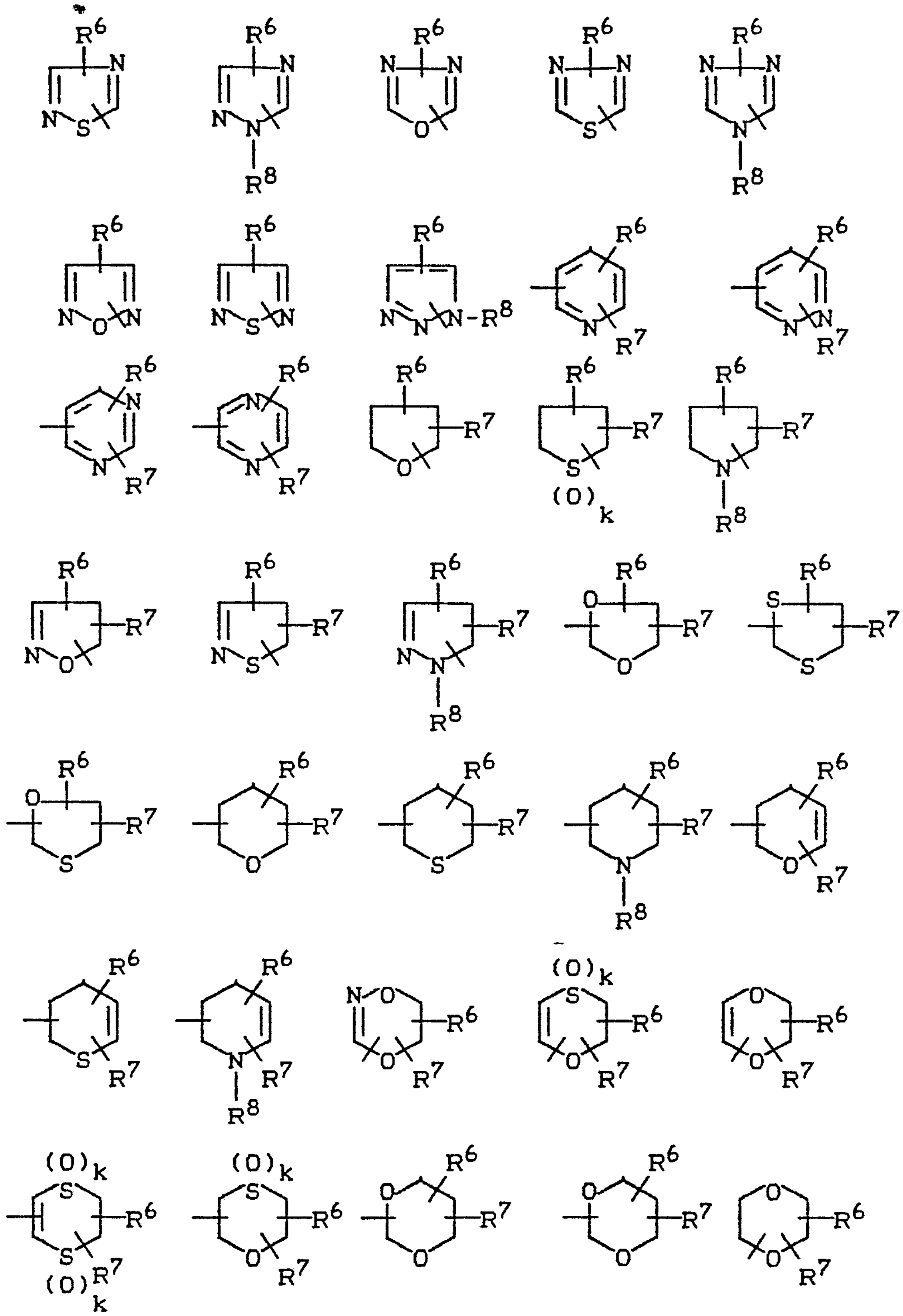

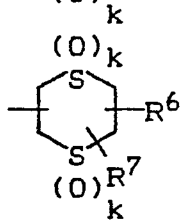

steht,

wobei in diesen Heterocyclen

$R^6$ und $R^7$ unabhängig voneinander für Wasserstoff, Chlor, Brom, Methyl, Methoxy, Methylthio oder Trifluormethyl stehen;

$R^8$ für Wasserstoff oder Methyl steht und

k für die ganzen Zahlen 0, 1 oder 2 steht.

5. 5-Acylamino-pyrazol-Derivate der Formel (I) gemäß Anspruch 1, in welcher

$R^1$ für Wasserstoff oder Nitro steht;

$R^2$ für Wasserstoff, Methyl, Ethyl, Allyl oder Propargyl steht;

$R^3$ für Wasserstoff steht;

$R^4$ für Wasserstoff steht;

X für Sauerstoff oder Schwefel steht;

n für die ganzen Zahlen 0 oder 1 steht;

Ar für gegebenenfalls ein- bis fünffach, gleich oder verschieden substituiertes Phenyl oder für gegebenenfalls ein- bis vierfach, gleich oder verschieden substituiertes 2-Pyridyl steht, wobei als Substituenten jeweils in Frage kommen:

Cyano, Nitro, Fluor, Chlor, Brom, Iod, Methyl, Ethyl, n- und i-Propyl, n-, i-, s-und t-Butyl, für Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentchlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^5$,

wobei

$R^5$ für Amino, Methylamino, Ethylamino, Dimethylamino, Diethylamino, Fluordichlormethyl, Difluormethyl, Tetrafluorethyl, Trichlorethyl, Trichlormethyl, Trifluormethyl, Methyl oder Ethyl steht

und

m für die ganzen Zahlen 0, 1 oder 2 steht,

Het für

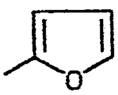 , 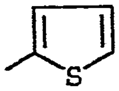 , 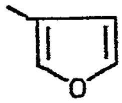 , 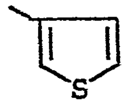 , 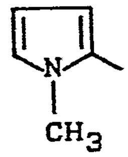 ,

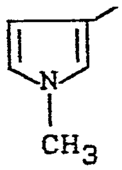 , 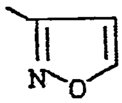 , 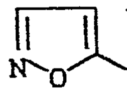 , 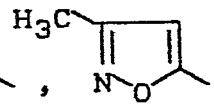 , 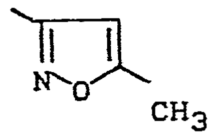 ,

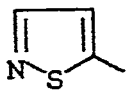 , 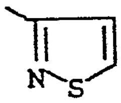 , 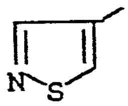 , 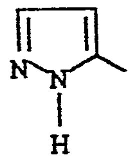 , 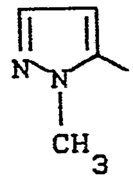

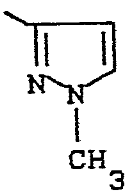 , 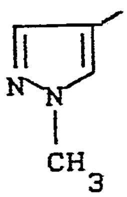 , 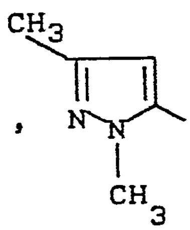 , 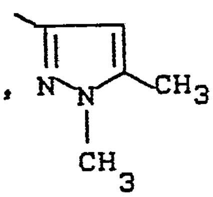 , 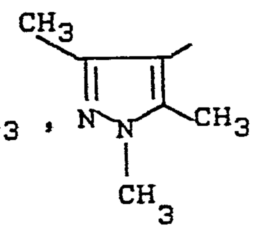

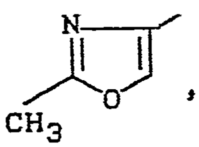 , 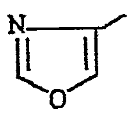 , 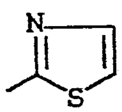 , 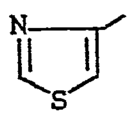 , 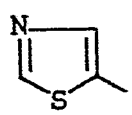 ,

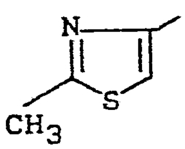 , 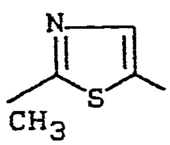 , 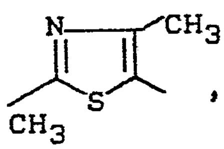 , 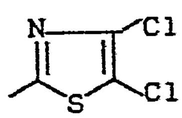 ,

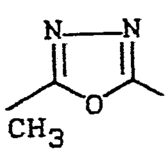 , 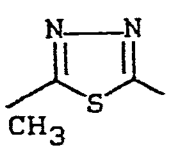 , 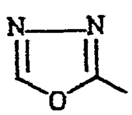 , 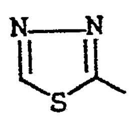 ,

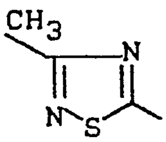 , 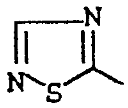 , 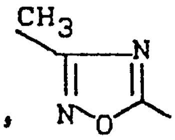 , 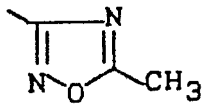 ,

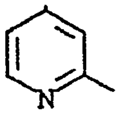 , 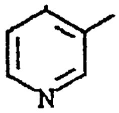 , 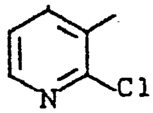 , 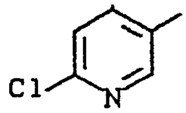 ,

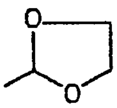 , 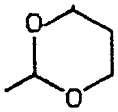 , 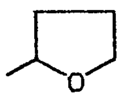 , 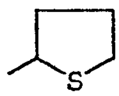 , 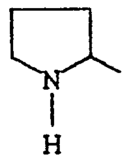 ,

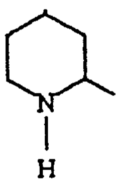 , 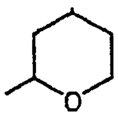 , 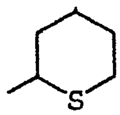 , 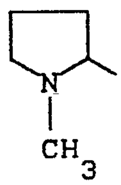 , 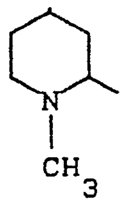 ,

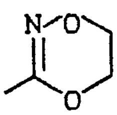 , 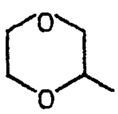 , 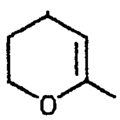 , 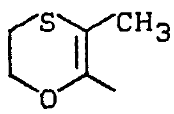 , 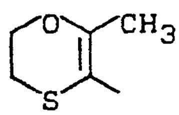 ,

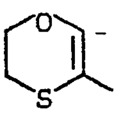 , 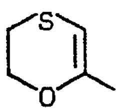 steht.

6. Verfahren zur Herstellung von 5-Acylamino-pyrazol-Derivaten der Formel (I)

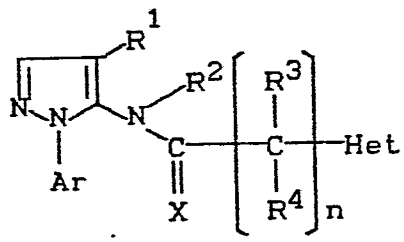

(I)

in welcher

$R^1$ für Wasserstoff, Halogen oder Nitro steht,

$R^2$ für Wasserstoff, Alkyl, Alkenyl, Alkinyl oder gegebenenfalls substituiertes Cycloalkyl steht,

$R^3$ für Wasserstoff oder Alkyl steht,

$R^4$ für Wasserstoff oder Alkyl steht,

X für Sauerstoff oder Schwefel steht,

n für die ganzen Zahlen 0, 1 oder 2 steht,

Ar für jeweils gegebenenfalls substituiertes Phenyl oder Pyridyl steht und

Het für einen gegebenenfalls substituierten 5- oder 6-gliedrigen, über ein Kohlenstoffatom verknüpften Heterocyclus steht,

dadurch gekennzeichnet, daß man

a) 5-Acylamino-pyrazol-Derivate der Formel (Ia)

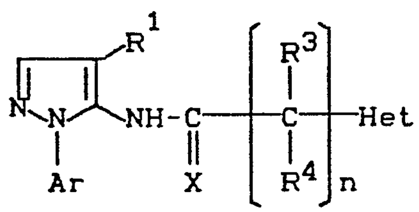

(Ia)

in welcher

$R^1$,$R^3$,$R^4$,X,Ar,Het und der Index n die oben angegebene Bedeutung haben,

erhält, wenn man 5-Amino-1-aryl-pyrazole der Formel (II)

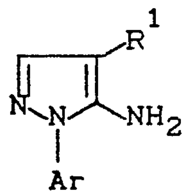

(II)

in welcher

$R^1$ und Ar die oben angegebene Bedeutung haben,

mit Acylierungsmitteln der Formel (III)

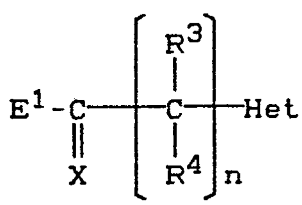

(III)

in welcher

X, $R^3$, $R^4$, Het und der Index n die oben angegebene Bedeutung haben und

$E^1$ für eine elektronenanziehende Abgangsgruppe steht,

gegebenenfalls in Gegenwart eines Verdünnungsmittels, gegebenenfalls in Gegenwart eines Säurebindemittels sowie gegebenenfalls in Gegenwart eines Katalysators umsetzt;

oder daß man

b) 5-Acylamino-pyrazol-Derivate der Formel (Ib)

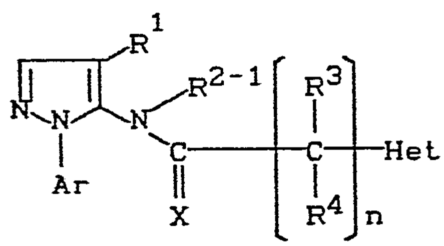

(Ib)

in welcher

$R^1$, $R^3$, $R^4$, X, Ar, Het und der Index n die oben angegebene Bedeutung haben und

$R^{2-1}$ für Alkyl, Alkenyl, Alkinyl oder gegebenenfalls substituiertes Cycloalkyl steht,

erhält, wenn man die nach Verfahren (a) erhältlichen 5-Acylamino-pyrazol-Derivate der Formel (Ia)

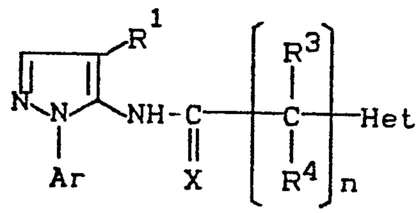

(Ia)

in welcher

$R^1$,$R^3$,$R^4$,X,Ar,Het und der Index n die oben angegebene Bedeutung haben,

mit Alkylierungsmitteln der Formel (IV)

$$E^2\text{-}R^{2-1} \qquad \text{(IV)}$$

in welcher

$R^{2-1}$ die oben angegebene Bedeutung hat und

$E^2$ für Halogen, für gegebenenfalls substituiertes Alkoxysulfonyloxy oder für gegebenenfalls substituiertes Arylsulfonyloxy steht,

gegebenenfalls in Gegenwart eines Verdünnungsmittel und gegebenenfalls in Gegenwart eines Säurebindemittels sowie gegebenenfalls in Gegenwart eines Katalysators umsetzt;
oder daß man

c) 5-Acylamino-pyrazol-Derivate der Formel (Ic)

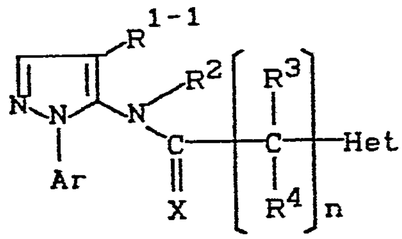

(Ic)

in welcher

$R^2$,$R^3$,$R^4$,X,Ar,Het und der Index n die oben angegebene Bedeutung haben und

$R^{1-1}$ für Halogen oder Nitro steht,

erhält, wenn man die mit Hilfe der Verfahren (a) oder (b) erhältlichen 5-Acylamino-pyrazol-Derivate der Formel (Id)

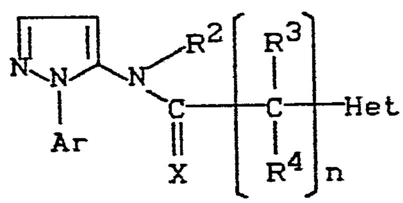

(Id)

in welcher

$R^2$,$R^3$,$R^4$,X,Ar,Het und der Index n die oben angegebene Bedeutung haben,

mit Halogenierungs- oder Nitrierungsmitteln der Formel (V)

$$R^{1-1}\text{-}E^3 \qquad (V)$$

in welcher

$R^{1-1}$ die oben angegebene Bedeutung hat und

$E^3$ für eine elektronenanziehende Abgangsgruppe steht,

gegebenenfalls in Gegenwart eines Verdünnungsmittels und gegebenenfalls in Gegenwart eines Katalysators oder Reaktionshilfsmittels umsetzt.

7. Herbizide und pflanzenwuchsregulierende Mittel, gekennzeichnet durch einen Gehalt an mindestens einem 5-Acylamino-pyrazol-Derivat der Formel (I) gemäß den Ansprüchen 1 bis 6.

8. Verfahren zur Bekämpfung von Unkräutern, dadurch gekennzeichnet, daß man 5-Acylamino-pyrazol-Derivate der Formel (I) gemäß den Ansprüchen 1 bis 6 auf die Unkräuter und/oder ihren Lebensraum einwirken läßt.

9. Verwendung von 5-Acylamino-pyrazol-Derivaten der Formel (I) gemäß den Ansprüchen 1 bis 6 zur Bekämpfung von Unkräutern und als Pflanzenwachstumsregulatoren.

10. Verfahren zur Herstellung von herbiziden und pflanzenwuchsregulierenden Mitteln, dadurch gekennzeichnet, daß man 5-Acylamino-pyrazol-Derivate der Formel (I) gemäß den Ansprüchen 1 bis 6 mit Streckmitteln und/oder oberflächenaktiven Substanzen vermischt.

0242573

Europäisches Patentamt

# EUROPÄISCHER RECHERCHENBERICHT

Nummer der Anmeldung

EP 87 10 3584

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
| A | DE-A-3 213 575 (SHOWA DENKO K.K.)<br>--- | | C 07 D 405/12<br>C 07 B 409/12<br>C 07 D 403/12<br>C 07 D 413/12<br>C 07 D 417/12<br>C 07 D 401/12<br>C 07 D 411/12<br>A 01 N 43/56<br>A 01 N 43/08<br>A 01 N 43/10<br>A 01 N 43/36<br>A 01 N 43/28<br>A 01 N 43/16<br>A 01 N 43/80<br>A 01 N 43/78<br>A 01 N 43/40<br>A 01 N 43/32 |
| A | FR-M- 4 000 (WINCKLER et al.)<br>--- | | |
| A | EP-A-0 154 115 (BAYER AG)<br>----- | | RECHERCHIERTE SACHGEBIETE (Int. Cl.4) |
| | | | C 07 D 405/00<br>C 07 D 409/00<br>C 07 D 403/00<br>C 07 D 413/00<br>C 07 D 417/00<br>C 07 D 401/00<br>C 07 D 411/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 29-06-1987 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82